# EUROPEAN PATENT SPECIFICATION

(11) **EP 3 880 285 B1**
(45) Date of publication and mention of the grant of the patent: **21.02.2024**
(21) Application number: 19883549.8
(22) Date of filing: 13.11.2019
(51) Int. Cl.: A61M 25/00, A61M 25/01, A61M 39/06

(54) **MEDICAL TOOL POSITIONING DEVICES**
VORRICHTUNGEN ZUR POSITIONIERUNG VON MEDIZINISCHEN WERKZEUGEN
DISPOSITIFS DE POSITIONNEMENT D'OUTILS MÉDICAUX

(30) Priority: 13.11.2018 US 201862760784 P
(43) Date of publication of application: 22.09.2021
(73) Proprietor: NuVera Medical, Inc., Los Gatos CA 95032 (US)
(72) Inventor: MIXTER, Colin, Los Gatos, CA 95032 (US); BITOUN, Marc, Los Gatos, CA 95032 (US); SAUL, Tom, Los Gatos, CA 95032 (US); SCHAER, Alan, Los Gatos, CA 95032 (US)
(74) Representative: Carpmaels & Ransford LLP
(86) International application number: PCT/US2019/061228
(87) International publication number: WO 2020/102389

(56) References cited:
- US-A- 4 817 613
- US-A1- 2004 176 740
- US-A1- 2012 277 671
- US-A1- 2012 277 671
- US-A1- 2017 189 646
- US-A1- 2018 279 994
- US-A1- 2018 279 994
- US-B1- 6 551 284
- US-B2- 7 402 151
- US-B2- 9 427 551

## Description

The disclosure herein may be related to disclosure in PCT Publication WO2018/017717 and US 2018/0279994 A1.

### BACKGROUND

A wide variety of intravascular medical devices are known. Improved systems, devices, and methods that facilitate better control, positioning, and usability of medical devices are needed.
US2012/277671A1 describes a dual braided catheter shaft including an inner helical braid and outer helical braid that encapsulate an axially extending steering wire there between. In one embodiment, the shaft includes an inner polymer jacket, an inner braid formed on the inner jacket, a steering wire disposed along an outside surface of the inner braid, an outer braid formed over the inner braid and steering wire assembly and an outer jacket formed on the outer braid. The braiding parameters of the inner and outer braids can be varied along the length of the catheter to provide varying mechanical properties. US2004/176740A1 describes a multiple braid exterior tube having a composite structure which includes an inner tubular layer, reinforcing layers and a polymer matrix layer. The exterior tube is formed with polymeric materials and metallic reinforcing braiding configured to provide greater tensile strength and stiffness. The exterior tube is used for a variety of medical devices such as a sheath component for intravascular devices and catheters.

### SUMMARY OF THE DISCLOSURE

The invention is defined in claim 1. Certain surgical methods are described with reference to the device of the present invention. Whilst no claim is directed to these methods per se, the device is capable of being used and is intended to be used in such methods.

One aspect of the disclosure is a medical device handle stabilizing apparatus, comprising: a body including an elongate and recessed handle guide, the recessed handle guide including a recessed channel configured to interface with an elongate handle portion of a medical device to provide stability to the handle portion in at least one direction, the body being biased to an upright at-rest position when positioned on a flattened surface, the recessed handle guide facing upward when the body is in the upright position.

One aspect of this disclosure is a medical device, comprising: an elongate handle housing; and an object capturing member secured relative to the handle housing, the object capturing member including a movable member that is movable relative to the handle housing from an open position to a closed position, wherein in the closed position the movable member is closer to a first portion of the handle than in the open position.

One aspect of the disclosure is a method of reducing movement of a medical device handle while in use, comprising: moving a movable member of a medical device handle from a closed to an open position relative to a handle housing of the medical device handle; positioning an article of clothing or garment between the moveable member and a second portion of the medical device handle; and moving the movable member to the closed position while capturing the article of clothing or garment to help stabilize the medical device handle.

One aspect of this disclosure is a medical device, comprising: an elongate handle housing; and a movable handle housing stabilizing member that extends away from the elongate handle housing, the stabilizing member having at least one surface that is adapted to be moved relative to the elongate handle housing to interact with an adjacent object and stabilize the elongate handle housing relative to the adjacent object.

One aspect of the disclosure is a medical device, comprising: an elongate handle housing; and one or more actuatable inputs that are in operative communication with a cable extending proximally from the elongate handle housing, a proximal region of the cable including a coupler that is adapted to be coupled to an external imaging console.

One aspect of the disclosure is a computer executable method stored on an external medical imaging console, comprising: receiving as input information indicative of an actuation of an input on an elongate medical device handle housing; and causing an image being displayed on the external medical imaging console to be modified.

One aspect of the disclosure is an elongate medical device sized and configured for intravascular delivery within a subject, comprising: a deflectable shaft extending distally from a handle portion of the medical device, the deflectable shaft including first and second braided layers each of which includes one or more strands, one of the first and second braided layer disposed radially within the other and co-axial with the other, and a flexible polymeric layers, the first and second braided layers at least partially embedded in the flexible polymer layers, the first braided layer having a first strand construction and the second braided layer having a second strand construction such that the first braided layer is better at transmitting torque alone than the second braided layer alone, and such that second braided layer is more resistant to kinking alone than the first braided layer alone, the flexible shaft better at transmitting torque compared to a second flexible shaft with the second braided layer and without the first braided layer, the flexible shaft more resistant to kinking than a third flexible shaft with the first braided layer but without the second braided layer.

One aspect of the disclosure is a medical device, comprising: an outer shaft and an inner shaft disposed within the outer shaft, at least one of the outer shaft and the inner shaft axially moveable relative to the other; and a flexible seal secured to at least one of the inner shaft or the outer shaft, the flexible seal extending outside of at least a portion of the inner shaft to seal off a volume of space radially adjacent the inner shaft from the ingress of fluid.

### BRIEF DESCRIPTION OF THE DRAWINGS

Figure 1A illustrates an exemplary embodiment of a system that includes steering and a medical device.
Figure 1B illustrates a cross section A-A of the steering and device portion of the medical device of Figure 1A.
Figure 2 illustrates an exemplary system that includes a handle assembly with a plurality of actuators, a steerable sheath and medical tool.
Figures 3Ai, 3Aii and 3Aiii illustrate an exemplary steerable shaft with pull wires.
Figures 3Bi and 3Bii illustrate an exemplary steerable shaft with pull wires.
Figures 3Ci and 3Cii illustrate an exemplary steerable shaft with pull wires.
Figures 3Di - 3Diiii illustrate an exemplary steerable shaft with pull wires.
Figure 3E illustrates an exemplary steerable shaft with one or more pull wires circumferentially interwoven into braid wires of the shaft.
Figure 4 illustrates an exemplary portion of an exemplary system that includes a bundle.
Figure 5 illustrates an exemplary proximal end of a medical tool, the tool including a conductor bundle that extends into a proximal connector within which is housed a printed circuit board (PCB).
Figure 6A illustrates a portion of an exemplary medical tool that includes a flexible circuit strip.
Figure 6B illustrates an exemplary proximal portion of a strip.
Figure 6C illustrates a detailed view of an exemplary proximal portion of a strip.
Figure 6D illustrates an end view of an exemplary flex strip.
Figure 6E illustrates an exemplary stack of flex strips.
Figure 6F illustrates an exemplary stack of flex strips and ground and shield strips.
Figure 6G illustrates an exemplary bundle including a tubing material around a stack of strips and shield and ground strips.
Figure 7 illustrates an integrated system of the steerable sheath and medical tool wherein the system is connected to a console via a connector cable.
Figures 8A and 8B illustrate an exemplary handle assembly that can be used with any of the inner and outer elongate bodies or shafts herein.
Figure 9A illustrates a portion of an exemplary inner elongate body.
Figure 9B illustrates a portion of an exemplary outer elongate body.
Figure 9C illustrates a portion of an exemplary medical device including the elongate bodies from figures 9A and 9B.
Figure 9D illustrates a section of the device in the deflectable portion from figure 9C.
Figure 10A illustrates a portion of an exemplary handle assembly.
Figure 10B is an exploded view illustrating an exemplary outer elongate body movement subassembly.
Figure 10C illustrates a side sectional view of the handle assembly from figure 57A.
Figure 11 illustrates an exemplary handle assembly that includes rotation indicators for first and second actuators.
Figure 12A illustrates an exemplary handle stabilization member.
Figure 12B illustrate an exemplary handle assembly, which is configured to be used with the exemplary handle stabilization member in figure 61A.
Figure 13 illustrates an exemplary handle stabilization body.
Figure 14 illustrates an exemplary handle with an object capture mechanism to reduce movement of the handle.
Figure 15 illustrates an exemplary handle with an object capture mechanism to reduce movement of the handle.
Figure 16 illustrates an exemplary handle with an object capture mechanism to reduce movement of the handle.
Figure 17 illustrates an exemplary handle with an object capture mechanism to reduce movement of the handle.
Figure 18 illustrates an example of a handle stabilization member.
Figure 19 illustrates an example of a handle stabilization member.
Figure 20 illustrates an example of a medical device that includes an elongate handle housing and one or more actuatable inputs.
Figures 21A, 21B, and 21B illustrate an exemplary braid construction for a medical shaft, the medical shaft including first and second braid layers.
Figure 22 illustrates an exemplary medical device with a flexible seal for reducing the inflow or fluid into a portion of the medical device.
Figures 23A, 23B, and 23C illustrate an exemplary medical device with a flexible seal for reducing the inflow or fluid into a portion of the medical device.
Figures 24A and 24B illustrate an exemplary medical device with a flexible seal for reducing the inflow or fluid into a portion of the medical device.
Figure 25 illustrates an exemplary medical device with a flexible seal for reducing the inflow or fluid into a portion of the medical device.
Figure 26 illustrates an exemplary medical device with a flexible seal for reducing the inflow or fluid into a portion of the medical device.
Figure 27 illustrates an exemplary proximal region of a medical device, including an exemplary seal therein.
Figure 28 illustrates an exemplary proximal region of a medical device, including an exemplary seal therein.
Figure 29 illustrates an exemplary proximal region of a medical device with a recess formed therein that is configured to be difficult to clean.
Figure 30 illustrates an exemplary proximal region of a medical device, including an exemplary handle housing with one or more features (e.g., protrusions) configured to compress a seal mechanism.

### DETAILED DESCRIPTION

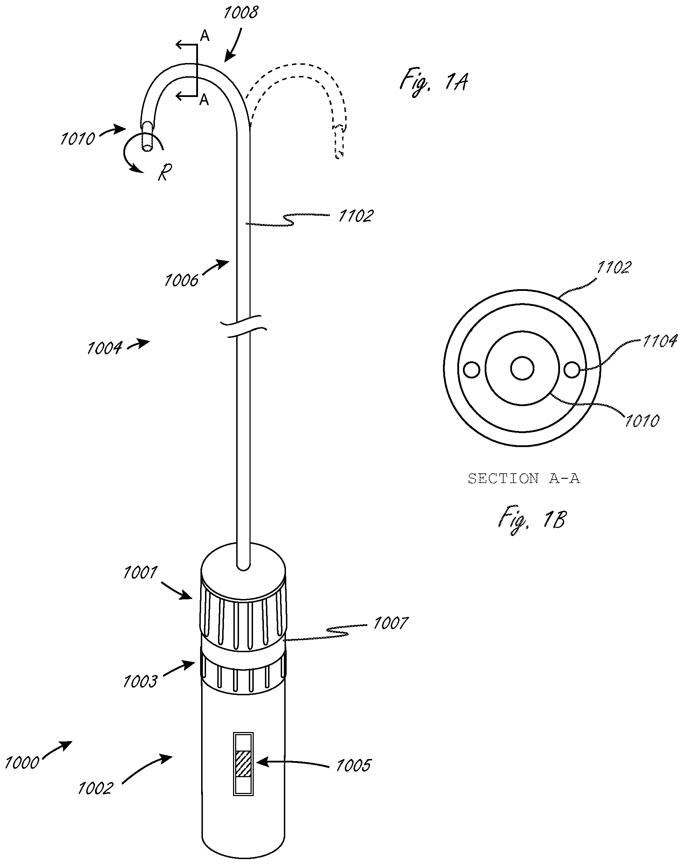

Figure 1A illustrates an exemplary embodiment of a system that integrates steering and a medical device. System 1000 includes handle assembly 1002 and steering and medical device portion 1004. Steering and medical device portion 1004 includes a proximal portion 1006 and steerable portion 1008. The system is adapted so that handle assembly 1002 can be actuated to cause steering of the steerable portion 1008, and optionally can be further actuated to cause movement of medical device 1010 relative to steering and medical device portion 1004. In this exemplary embodiment, handle assembly 1002 includes first actuator 1001, second actuator 1003, and third actuator 1005. First actuator 1001 is adapted to be actuated (in this example rotated) relative to handle body 1007 to cause the steering of steerable portion 1008, and specifically steering outer sheath 1102. Steerable portion 1008 in this embodiment can be steered, or bent, into the configuration shown in Figure 1A in solid lines, and can also be steered into the configuration shown in dashed lines, or anywhere in between, and in some embodiments the opposite steering function is limited to simply straightening the shaft from an initial bent configuration, such as the solid line bent configuration in Figure 1A. The term "steer" in this disclosure means to deflect or bend, optionally via actuation of at least one pull wire, but in some instances the term can include shaft rotation (torqueing) and axial movement. The term "pull wire" herein refers to any element that may transmit a tensile force from the proximal end of the device to the distal end region. Pull wires may be comprised of metal wire such as stainless steel or nickel titanium, either solid or stranded/braided, or it may be comprised of a polymer such as aramid fiber (Kevlar^{®}), polyethylene, ptfe, eptfe, etc., preferably stranded/braided, but also in monofilament form. In a preferred embodiment, the pull wire is constructed from an aramid fiber bundle having four 50 denier multifilament (approximately 25 filaments) threads braided together at a high picks per inch (1 inch = 25.4 mm) The wire cross-sectional diameter is typically in the .005"-.012" range, more preferably .008"-.010", although braided or stranded wire may flatten or ovalize in the device lumen. The preferred construction embodiments are believed to provide optimized strength and wear resistance for the size necessary to keep the shaft diameters to a minimum. Optional second actuator 1003 is adapted to be actuated relative to handle body 1007 (in this example rotated) to cause rotation of medical tool 1010 relative to shaft 1102 (labeled as rotation movement "R"), and optional actuator 1005 is adapted to be actuated relative to handle body 1007 (in this example axially) to cause axial (distal-proximal) movement of medical device 1010 relative the outer sheath 1102. Proximal portion 1006 is not configured to bend significantly when steerable portion 1008 is steered (bent/deflected), although the proximal portion may flex and bend to conform to the anatomy within which it is used. In many embodiments, this is accomplished by constructing the steerable portion 1008 from a softer or less rigid material and/or composite construction than the proximal portion 1006.

The embodiment shown in Figure 1A is an example of an apparatus that includes an integrated handle assembly that is in operable communication with both a steerable outer shaft and an inner medical tool. The handle assembly is integrated in that it is assembled and constructed to be in operable communication with the outer shaft and the inner medical tool prior to packaging and use. "Integrated" as that term is used in the context of an integrated handle assembly refers to a handle assembly in which at least one part of the handle assembly has to be broken or taken apart before the medical tool can be removed from within the outer shaft.

Figure 1B illustrates an exemplary cross section A-A (shown in Fig. 1A) of the steering and device portion 1004, and specifically in the steerable portion 1008. In this embodiment medical device 1010 is sized and configured to be disposed within a steerable sheath. The steerable sheath includes an outer shaft 1102 and a set of pull wires 1104, which are axially fixed in a distal region of steerable portion 1008.

The medical tool in Figures 1A and 1B can be, for example, any medical tool herein, such as an ultrasound tool. When "ultrasound probe" is used herein, it generally refers to an elongate tool that includes at least one ultrasound transducer and one or more conductive elements that electrically connect the at least one ultrasound transducer to a proximal region of the elongate tool. A proximal region of the ultrasound probe includes, or is modified to include, at least one proximal contact, which is in electrical communication with the at least one ultrasound transducer, and which can be put into electrical communication with, optionally via attachment to, an electrical contact on another device, cable, or connector.

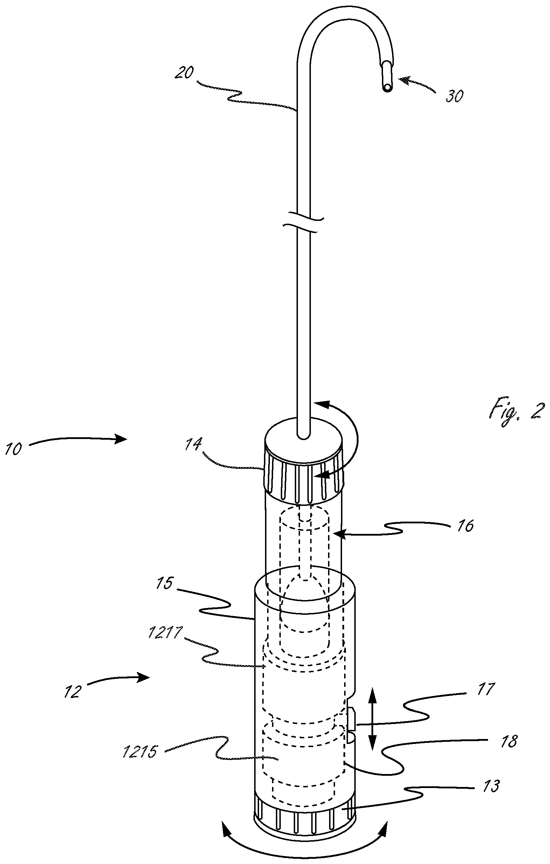

Figure 2 illustrates an exemplary system 10 that is adapted to function similarly to the system in figures 1A and 1B, and also illustrates exemplary internal components of handle assembly 12 (internal components shown as dashed lines). Handle assembly 12 is integrated and in operable communication with outer steerable shaft 20 and medical tool 30. Handle assembly 12 includes actuator 14 that is adapted to, when actuated relative to handle body 15, cause steering of steerable shaft 20. Actuator 14 is in operable communication with steerable shaft 20 via steering control 16 disposed in handle assembly 12. Medical tool 30 includes a proximal portion 18 disposed within and incorporated into handle assembly 12. Actuator 13 is in operable communication with medical tool 30, and actuation of actuator 13 (in this example rotation) relative to handle body 15, causes rotation of medical tool 30 relative to outer shaft 20 via rotation control 1215. Optional third actuator 17 is also in operable communication with medical tool 30, and is adapted to be actuated, in this embodiment, axially (relative to handle body 15), to cause axial movement of medical tool 30 relative to outer steerable shaft 20 via axial control 1217.

The medical tool in Figure 2 can be, for example, any medical tool herein, such as an ultrasound tool.

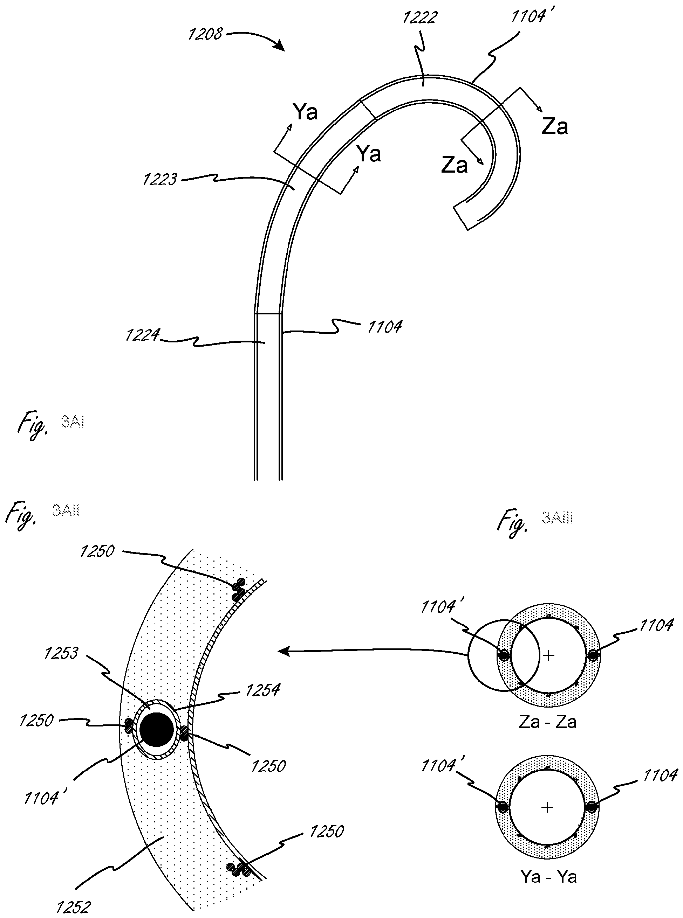

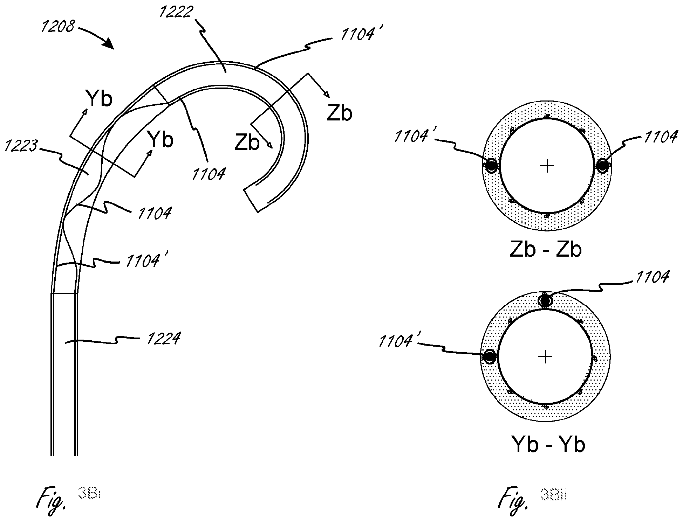

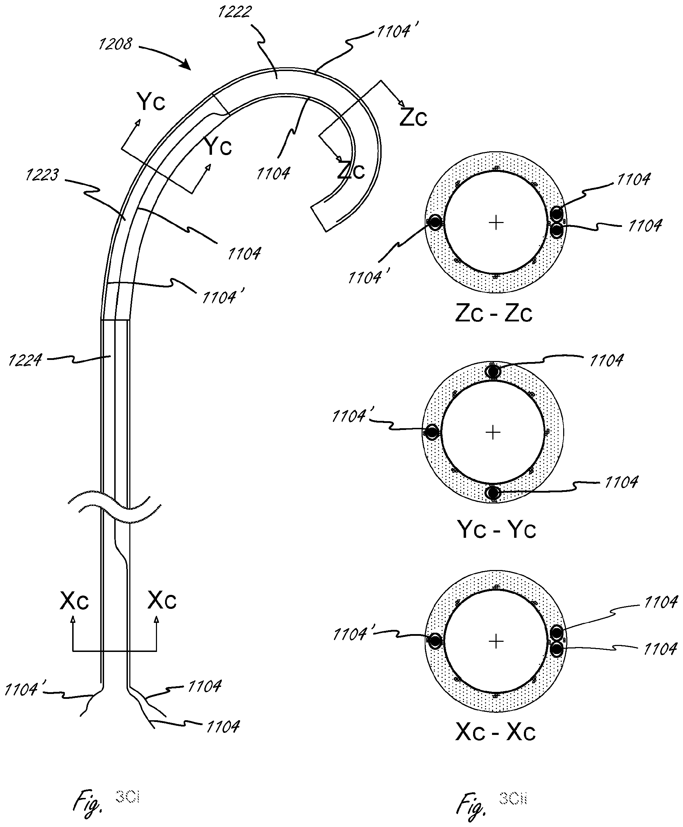

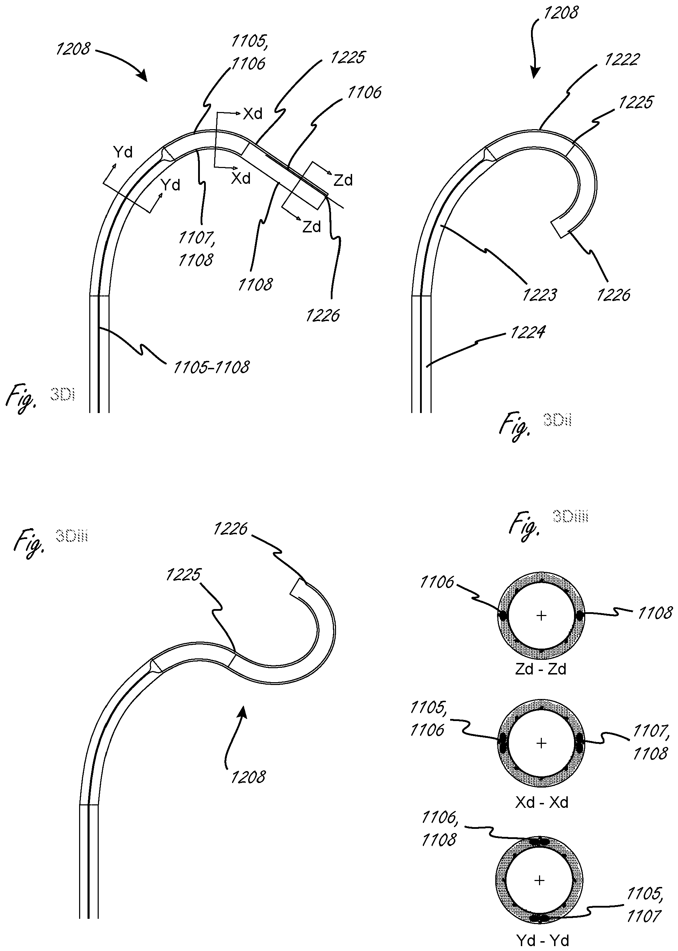

Figures 3A-3E represent exemplary embodiments of a distal region of the sheath portion 1208 of steerable sheath 1202 in system 1200. For simplicity, the illustrated cross-sections show only the outer sheath 1208 and not the inner tool 1212. The outer sheath 1208 preferably has a composite construction to improve torque transmission applied to the outside of the shaft from the proximal end, or to resist torque forces applied to it from within the shaft, such as from tool 1212. As illustrated in Figure 3Ai-iii, in order to form the composite, multiple braid elements 1250, preferably formed from metal wire (round, pairs of round, or ribbon shaped) and/or multiple fibers (e.g., aramid or nylon), may be braided directly over a thin wall (e.g., .0010" ± .0005") lubricious liner tube 1251, such as a PTFE or FEP material. A thermoplastic polymer 1252 (such as Pebax in a range of durometers from 25D-72D, or nylon, or other common catheter materials) may be laminated with heat using heat shrink tubing (such as FEP) to reflow the polymer over the braid elements 1250 and liner tube 1251 to form a uniform member. The thermoplastic polymer 1252 may also have radiopaque compounds that include materials such as bismuth, barium sulfate, or tungsten in order that the tip of the sheath be visible to the user under fluoroscopy. In the embodiment of Figure 3Ai-iii, the pull wire 1104 is preferably parallel to the central access in the steerable (deflectable) portion 1222 of the sheath and also preferably provided in a lumen 1253 created within the wall of the steerable sheath 1208. This lumen may be created during the thermoplastic polymer tubing extrusion process or during a shaft heat lamination fusing process with the aid of a removable mandrel. The pull wire lumen 1253 may further be created by incorporating a pull wire tube 1254, preferably temporarily supported by a removable mandrel, within the wall. The removable mandrel may also be placed alongside the pull line 1104 or 1104' during the fusing process, resulting in a somewhat ovalized lumen 1253 within which a fiber pull wire may be allowed to flatten into, allowing space for free movement of the pull wire. The tube 1254 may include PTFE, FEP, polyimide, or another material which maintains its wall integrity during a heat lamination process up to approximately 500 °F. The tube is preferably surrounded and supported by the thermoplastic polymer 1252 which is preferably heat laminated against the tube. In another embodiment, the pull wire lumen, preferably comprising the pull wire tube, is incorporated within the weave of the braid elements 1250. For example, braid elements 1250 running in one direction would pass under the pull wire lumen, while those running in the opposite direction would pass over the pull wire lumen. The braid reinforcement provides a more dimensionally stable lumen during catheter manipulations and also helps assure the straightness of the lumen as needed. Proximal to the steerable portion, the pull wire may continue proximally parallel to the central axis on the same side of the outer sheath 1208, such as is illustrated in Figure 3Ai-iii. In this embodiment and others that follow, an additional pull wire 1104' within an additional pull wire lumen routed within the wall of sheath 1208, up through the steerable portion 1222, may be required to straighten the steerable portion of the device. This straightening pull wire 1104' is preferably routed within steerable portion 1222 on the side opposite from the pull wire(s) 1104 used for steering (deflection) in the steerable portion 1222. In another embodiment, not shown, two lumens and two straightening pull wires 1104' could be used, essentially mirroring the paired 1104 pull wire configuration. These straightening wires could also be constructed to allow deflection in the opposite direction by tensioning a greater distance (beyond just straightening) within the handle.

During use, a portion 1223 of the distal catheter just proximal to the steerable (deflectable) portion 1222 may be forced to conform to a curve based on the constraints of the anatomy in which it is used. For a specific embodiment where the device is advanced into the heart chambers from a groin access, the portion 1223 forced into a curve is expected to range from 5 to 25 cm in length. During rotation of the sheath shaft 1208 from the proximal end, torque is transmitted through this distal curved region 1223 to the catheter tip. A non-uniform cross section and/or tension of the device in this region 1223 may induce a tendency for the shaft to build up and suddenly release torque, causing a "whip" or sudden jerk in rotation as it is torqued. To minimize the potential for whip, it is optional to distribute the pull wire tension and construction material around the surface of the curved region 1223. In one embodiment, such as is illustrated in Figure 3Bi-iii, the pull wire 1104 may spiral around the central axis of the sheath in at least the curved region 1223 proximal to portion 1222. The pull wire of this embodiment may make a full circumferential wrap over approximately 10 cm of length, with this value ranging 5-15 cm. The spiral may only need to be present in the curved region 1223, continuing straight proximally thereafter through proximal portion 1224 (similar to 1006), which may minimize the friction in the pull wire lumen and the associated pull wire force required to steer (deflect) the steerable portion 1222. The spiral may also make a minimum of one turn before continuing straight, or spiral the full length of the shaft. In another embodiment to minimize whip, it may only be necessary to distribute the pull wire tension to opposite sides of the shaft. As illustrated in Figure 3Ci-ii, deflection of the steerable section 1222 is accomplished with two parallel pull wires 1104 positioned adjacent one another on the same side of the sheath 1208. In the curved region 1223 and proximal portion 1224 (similar to 1006) proximal to the steerable section 1222, the pull wires are routed to opposite sides of the shaft, each 90° from the position in the steerable section 1222, to distribute the tension more evenly. While it is preferable to actuate the two parallel pull wires at the same time with equal force with the handle actuator, in other embodiments, a differential in force could be applied to steer the tip to one side or the other of the plane formed when the two are actuated with equal force. In other embodiments, any plurality of pull wires could be routed in the same configuration as illustrated in Figures 3B or Figure 3C, with the multiple proximal pull wires distributed uniformly around the shaft circumference. Also, as illustrated in Figure 3Ci-ii, the pull wires 1104 may be routed proximally along the opposite sides of the shaft for most of the shaft proximal portion 1124 length, but preferably brought back together adjacent one another near the proximal end portion of the shaft to allow the wires to exit the same side of the proximal shaft together to facilitate them being secured together to a handle component for simultaneous actuation tension.

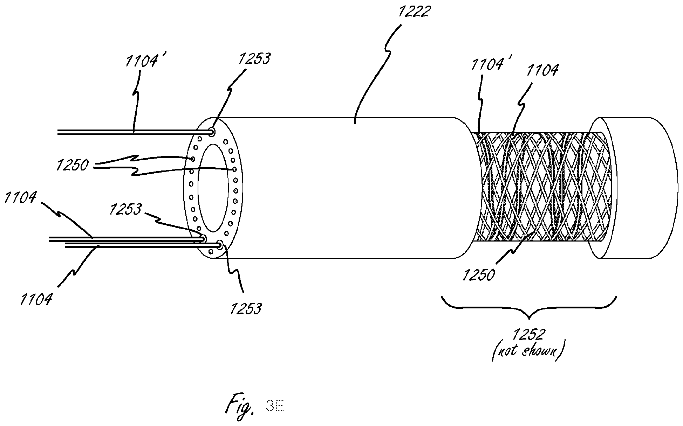

Figures 3Di-iv illustrate another embodiment of the distal region of catheter with construction similar to that previously described, but instead configured to provide a distal steerable portion 1222 which can be deflected into two different directions. As illustrated, a two pairs of pull wires 1105/1107 and 1106/1108 are along the proximal shaft region 1224 and curved region 1223. This is similar to Figure 3Ai-iii, except that the wires are paired on each side of the shaft. The routing could also be spiraled as in Figure 3Bi-ii, or other configurations discussed. Within distal steerable portion 1222, the wires are routed 90° from the proximal portions, although other angles are contemplated. At a junction 1225 within 1222 one or more of the pull wires (e.g., 1105 and 1107) may be terminated and anchored to the shaft, with the remaining pull wires (e.g., 1106 and 1108) continuing to a more distal tip location 1226 where they are anchored. This configuration allows independent actuation of pull wires terminated at 1225 and 1226 such that different shapes may be created during actuation. Figure 3Dii shows both lines 1107 and 1108 tensioned to create a variable curve in the same direction. Figure 3Diii shows lines 1107 and 1106 tensioned to create an "S" curve. Other configurations are also possible.

The pull wires (such as 1104 and 1104') must be terminated at their distal end in a manner that reliably affixes them to the wall of the distal steerable shaft portion 1222, such that they do not break or pull free under repeated applications of tension. In a preferred embodiment, shown in Figure 3E, the pull wires 1104 and 1104', upon exiting the distal pull wire lumen 1253, are circumferentially interwoven into the braid wires 1250 of the distal shaft 1222 (shown without the thermoplastic polymer 1252). One or more of the pull wires 1104 or 1104' may also be additionally or instead wrapped and/or tied around the outside of the braid wires 1250 for additional securing. The braid wires 1250 may be then trimmed distal to the securing point, with the interwoven and/or wrapped pull wires preventing the braid wires from expanding and/or unraveling. Additional adhesives such as UV cured or cyanoacrylates may also be used to secure the pull wires to the braid wires. The weave and/or wrap of the pull wires and braid wires is then laminated with a thermoplastic polymer which melts within the space around the wires and cools to secure them in place. The thermoplastic polymer may also have radiopaque compounds that include materials such as bismuth, barium sulfate, or tungsten in order that the tip of the sheath be visible to the user under fluoroscopy.

In additional embodiments, the tool 1212 may also or alternatively be constructed with one or more pull wires to deflect the tip in a manner similar to any of the previous embodiments described for the outer sheath 1208. In addition to routing the pull wires within the wall of the tubular member of the tool 1212, the pull wires could be routed next to the conductors inside the lumen of the tubular element 1212. Actuation of the pull wires could be from an actuator located in the proximal handle 1206. The distal shaft of tool 1212 may also be formed into a particular shape (e.g., an arc) such that it bends into the shape as it exits the tip of the steerable portion 1222 of outer sheath 1208. The stiffness of the distal shaft of tool 1212 is such that it does not substantially deform outer sheath 1208 while inside, but upon exiting is allowed to bend. The shape may be set by any one or combination of the following means: heat setting the polymeric material, using a moveable or fixed shaped stylet within the inner lumen of shaft 1212 or within a lumen within the wall of shaft 1212. Such a stylet could be round, oval, or rectangular in cross section, and be formed of stainless steel, nitinol, or a rigid polymer such as PEEK, Vestamid, or similar. The outer steerable sheath could alternatively be made to bend with a similar method as above, with or without additional pull wire deflection, and with or without additional shape or deflection of the distal portion of tool shaft 1212.

One aspect of the disclosure includes methods of disassociating at least a portion of the system from other components, optionally as part of a reposing process. In some embodiments the medical tool includes one or more electrical contacts that are coupled to other electrical contacts, which are in electrical communication with an energy console, and examples of consoles are known in the ultrasound art.

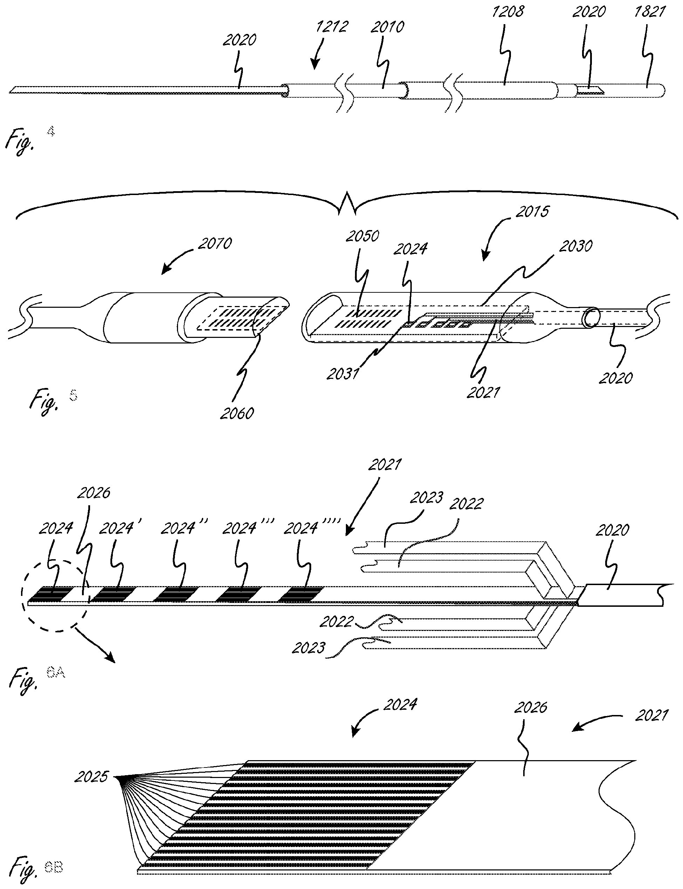

Figure 4 illustrates merely a portion of an exemplary medical tool, such as an ultrasound probe, that can be electrically coupled directly or indirectly to an energy console, such as an ultrasound console.

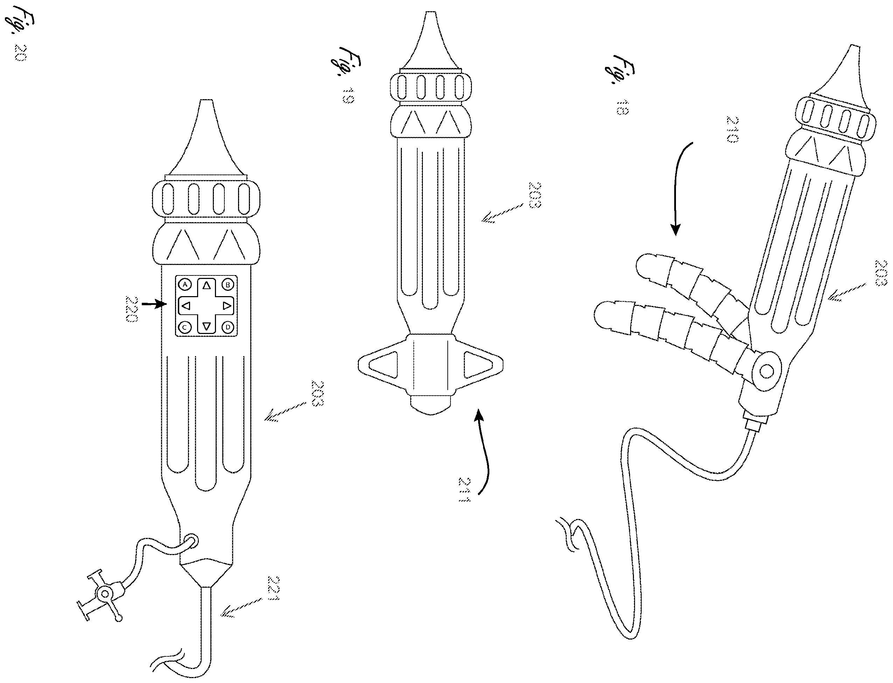

Reposing the device can involve disconnection of one or more proximal electrical contacts and moving the tool portion distally out of the distal end of the sheath portion. In this embodiment tool portion 1212 comprises at least a tool outer sheath or member 2010, distal working end 1821 (which can include at least one ultrasound transducer), and conductor bundle 2020. The conductor bundle 2020 extends from the distal working end 1821, through the tool outer member 2010 to a proximal connector (the connector and handle mechanism are not shown in Fig. 18 for clarity). In some embodiments the medical tool is used for ultrasound imaging, optionally where the distal working end 1821 comprises a two-dimensional (2D) array of piezo electric components mounted on an ASIC (application specific integrated circuit).

Figure 5 illustrates a merely exemplary proximal end of a medical device (the medical device is shown on the right), and in this embodiment the medical device is an ultrasound probe. The proximal end 2015 of the medical device is adapted to be electrically coupled to connector cable 270, which is directly or adapted to be indirectly electrically coupled to an energy console, such as an ultrasound energy console. As illustrated in Figure 5, conductor bundle 2020 extends from a distal region of the medical tool (distal region not shown) into a proximal connector 2015 within which is housed a rigid or flexible printed circuit board ("PCB") 2030. The connector bundle 2020 includes a plurality of contacts 2024 (examples of which are described below) that are attached to PCB board contacts 2031. Each individual trace from each contact 2031 is linked to individual exposed contacts 2050 on another portion, optionally more proximal, of the PCB. The individual PCB traces may also pass through other useful circuitry on the PCB. The exposed contacts 2050 are configured for a mechanical mating for electrical conduction to similar contacts 2060 on mating connector cable 2070, similar in concept to the proximal tool connector 1990 described previously, which links the tool 1204 to a user-interface console. Proximal connector 2015 can be incorporated into any of the systems, handles, steerable sheaths, medical tools, etc., herein.

Figures 6A and 6B illustrate an exemplary conductor strip (also referred to herein as a flexible circuit strip) 2021 that can be included in any of the conductor bundles herein. The embodiment in Figures 6A and 6B is an example of a conductor strip that can be included in bundle 2020 from Figures 4 and 5. The embodiment in Figures 6A and 6B can be incorporated into any other system herein.

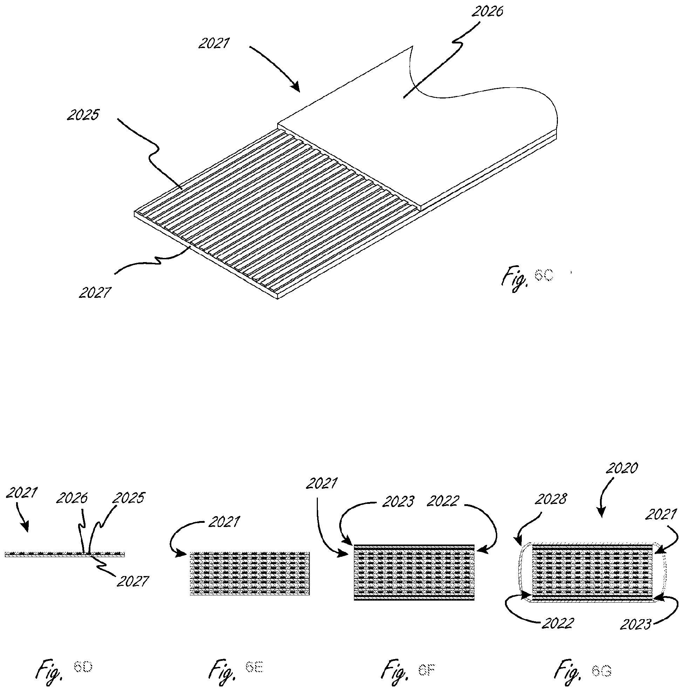

As shown in Figures 6A, 6B and 6G, conductor bundle 2020 comprises a plurality of flex circuit strips, including multi-trace strips 2021, as well as conductive strips for grounding 2022 and shielding 2023 (only a portion of which are shown). Each multi-trace strip comprises a plurality of conductive traces 2025, which can be seen clearly in Figures 6B, 6C and 6D. The number traces 2025 in Figures 6D-G is twelve, and the number of traces in Figures 6A-6C is sixteen, and they are both exemplary as to the number of traces 2025 that can be used. Each strip 2021 can be approximately .072" wide and .0022" thick, and can optionally comprise sixteen .0022" wide x about.0007" thick conductive (e.g., copper) traces, each spaced approximately .0022" apart. The traces are disposed on an insulating substrate layer 2027, such as a polyimide substrate, and the traces can be at least partly covered by a cover layer 2026, such as a photoimageable film cover ("PIC") layer or other dry film solder mask (DFSM) or other similar material. The cover layer generally extends along most of the bundle, except at discrete locations in proximal and distal regions for electrical coupling. In other embodiments, the strip 2021 is approximately .055" wide and comprises twelve conductive traces (see figures 6D-G). In other embodiments, the strip 2021 is approximately .037" wide and comprises eight copper conductive traces. The outer strips 2022 and 2023 used for grounding and shielding may have a similar construction and dimension except they can comprise a single full width strip of copper. As optimized for a 2D piezo array, a stack of approximately seven 16-trace strips 2021 would be required (or nine 12-trace, or fourteen 8-trace), along with one each of strips 2022 and 2023 on each side of the stack of multi-trace strips. Figure 6E illustrates a portion of an exemplary bundle 2020 with nine strips 2021 stacked together. Figure 6F illustrates a portion of the bundle that includes nine strips 2021 stacked, as well as ground strip 2022 and shield strip 2023 (only those on top are labeled). The complete bundle may optionally be held together with a, for example without limitation, about.001" wall thickness shrink tube, such as the tubing 2028 in Figure 6G. The flex circuit dimensions and number of traces discussed above are for a particular configuration of a piezo-electric array (and/or an ASIC controller thereof) and may be varied depending on how the number and size of array elements are optimized for the particular application.

The proximal end of each flex circuit strip has the conductive material (e.g., gold-plated copper) exposed over a length of approximately, for example, 3mm through removal of the cover layer 2026 at location 2024. Location 2024, and other exposed locations described herein, is generally referred to as a "contact." It is understood that when used in this context, the contact actually includes a plurality of separated conductive traces (such as shown in region location), each of which is adapted to be in electrical communication with its own corresponding conductive element. "Contact" is therefore not limited to mean only a single electrical connection between two conductive elements. While Figure 6A shows a plurality of exposed regions 2024, the embodiment in Figure 6A will first be described herein as if there is only one exposed region (i.e., region 2024 at the proximal end). The strip 2021 can be made to create an electrical connection to matching exposed contacts 2031, shown in Figures 6A-C, for conductive traces on the PCB 2030. In some embodiments, sixteen individual traces, sized and spaced to match sixteen traces in the multi-trace strip 2021, would be provided within a given contact 2031. An ACF (anisotropic conductive film), soldering, conductive adhesive, mechanical connection, or any combination of these may be used to achieve a suitable electrical connection (electrical coupling) between the strip traces and the PCB contacts.

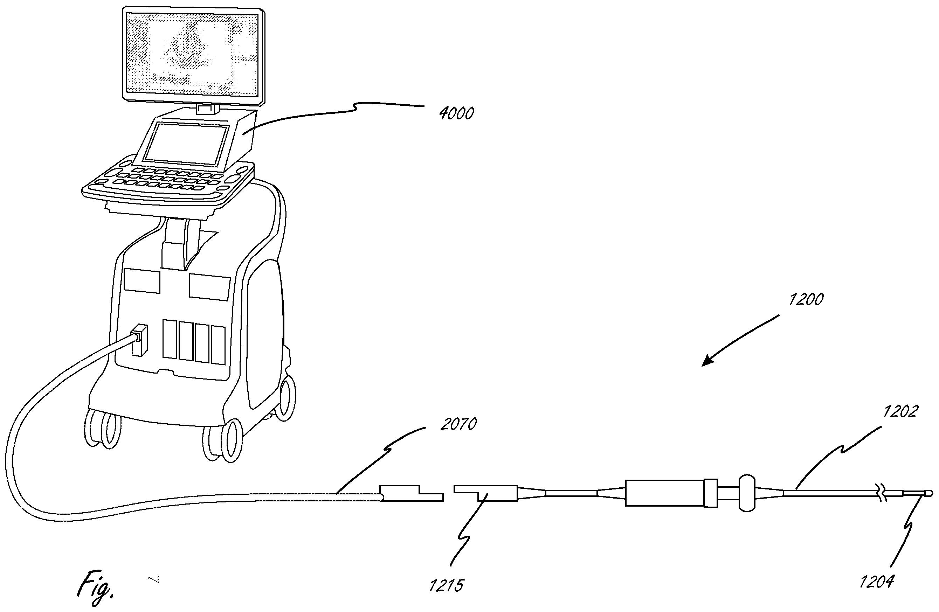

Figure 7 illustrates the integrated system 1200 of the steerable sheath 1202 and medical tool 1204 wherein the system 1200 is connected to console 4000 via the connector cable 2070. As previously described, such as for Figure 5, the tool 1204 comprises a proximal connector 2015 which forms a mating connection to cable 2070. As previously described, it is desirable to repose (e.g., reprocess and reuse) the system 1200. It is further desirable to ensure that the system is reposed only by the original manufacturer and not an unaffiliated third-party, and to ensure the device is only reused a specified number of times. To control the reposing process, a crypto-authentication chip (crypto-chip) is incorporated into the tool 1204, preferably on the PCB 2030, although other locations, such as within the steerable handle 1206, or within the tip 3000, are contemplated. The crypto-chip is programmable only by the original manufacturer who controls the authentication keys. The console 4000 to which the system 1200 is connected has a Trusted Platform Module (TPM) which also has the authentication keys. During use of the system 1200, the console 4000 is able to authenticate the system 1200 via the crypto-chip and as desired may read and write information to the chip (e.g., via an EEPROM feature). In any of the scenarios discussed, RFID chips, preferably encrypted, may be used to read and transmit data between the console, connector, and the device.

As used herein, "cleaning" can refer to any type of cleaning, such as without limitation: cleaning an interior of an outer shaft using a flushing system of cleaner and/or disinfectant and optionally mechanical scrubbing with small brushes; mechanical cleaning (e.g., wipes, brushes) an outer portion of an outer shaft and/or outer portion of a medical device shaft (e.g., ultrasound probe) with a cleaner/disinfectant, and optionally submerging the shaft in an ultrasound bath of cleaner/disinfectant for a specified period of time; and optical cleaning methods such as comprising using UV light. "Cleaning" as used here does not refer to a specific cleaning process, but rather refers to the general idea of cleaning an object.

The disclosure herein also includes methods of assembling or reassembling any of the subassemblies or assemblies herein, including any of the subassemblies within any of the handle assemblies herein. For example without limitation, the disclosure here includes methods of spooling one or more pull wires over a bearing surface in a spindle support and then around the spindle.

The methods herein also include manufacturing or constructing any of the individual components of any of the subassemblies or assemblies herein. For example, the disclosure includes methods of manufacturing handle shell components that have particular configurations (e.g., guides, walls, etc.) that can accommodate the internal parts that allow the assemblies or subassemblies herein to function as intended.

Regardless of the reference number with which they are labeled, any of the handle assemblies, medical tools, steerable sheaths, and electrical connections herein can be used together in a system in any combination with each other.

Any of the technology, including ultrasound and steering technology, in any of the following U.S. patent references may be incorporated into any of the medical tools, devices, systems, or methods of use thereof herein6100626, 6537217, 6559389, 7257051, 7297118, 7331927, 7338450, 7451650, 7451650, 7527591, 7527592, 7569015, 7621028, 7731516, 7740584, 7766833, 7783339, 7791252, 7791252, 7819802, 7824335, 7966058, 8057397, 8096951, 8207652, 8207652, 8213693, 8364242, 8428690, 8451155, 8527032, 8659212, 8721553, 8727993, 8742646, 8742646, 8776335, 8790262, 8933613, 8978216, 8989842, 9055883, 9439625, 9575165, 9639056, and 20080287783.

Any suitable disclosure above can be incorporated into any of the embodiments below. For example, aspects of devices, systems, and methods of manufacture and use are incorporated herein and can be incorporated into any of the embodiments below unless specifically indicated to the contrary.

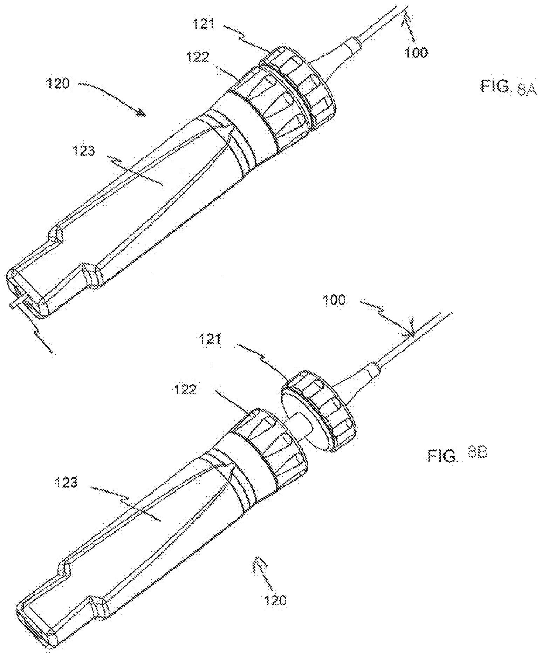

Figures 8A and 8B illustrate a merely exemplary handle assembly that can be in operable communication with elongate body 117 and elongate inner body 110. In this exemplary implementation, handle assembly 120 includes handle body 123 that has an outer surface that can be gripped by a user, first actuator 121, and second actuator 122. Actuator 121 can be in operable communication with elongate body 100, and actuator 122 can be in operable communication with elongate inner body 110. Actuator 121 is adapted to be both rotated and moved axially relative to handle body 123 (and relative to second actuator 122). This allows actuator 121 to cause axial movement of the medical tool 103 and rotation of the medical tool 103 relative to a distal end of elongate inner body 110. Second actuator 122 is adapted to be actuated (e.g., rotated in this embodiment) relative to handle body 123 to cause deflection of the elongate inner body 110. For example, the handle assembly can have internal components that interface with proximal ends of tensioning members (e.g. tensioning members 111, 112 and 116) such that actuation of actuator 122 tensions one or more tensioning members to cause deflection of the elongate inner body, and thus also the medical tool. In this embodiment actuator 121 is distal to actuator 122, but in other designs their relative positions could be reversed. Figure 8B shows handle assembly 120 after actuator 121 has been advanced distally relative to its position in figure 8A. This distal advancement causes the outer elongate body 100 to be advanced distally, and thus causes the medical tool to be advanced distally. Actuator 121 can similarly be retracted proximally relative to its position in figure 8B.

In other designs, actuator 121 could be in operable communication with elongate inner body 110 and actuator 122 can be in operable communication with elongate body 100.

Slack can be added to the one or tensioning members (e.g., pull wires) in the handle assembly to better allow the outer elongate body translate relative to the elongate inner body.

As described herein, the outer shaft can be moved axially relative to the inner deflectable shaft. The outer shaft can be constructed with sections of materials that vary in stiffness (e.g., durometer) along the length of at least a portion of the outer shaft. For example, a first portion that is distal to a second portion can have a lower durometer than the second portion. Because the outer shaft can be moved axially relative to the deflectable inner shaft, and because the stiffness of the outer shaft can vary along its length, the deflection, including the degree (or amount), of the overall device can be selectively controlled by controlling the axial position of the outer shaft (relative to the inner shaft). Axial movement of the outer shaft can thus selectively control deflection of the device. For example, a user (e.g., physician) can change or control where the bend occurs along the length of the device (measured from the distal end) by axially moving the outer shaft relative to the inner shaft. Additionally, for example, sections of varying stiffness in the outer shaft can allow for more or less deflection depending on the relative position of the outer shaft relative to the deflectable inner shaft. For example, deflecting the inner shaft at a region where the outer shaft has a relatively higher stiffness can result in less deflection than when the inner shaft is deflected at a region where the outer shaft has less stiffness.

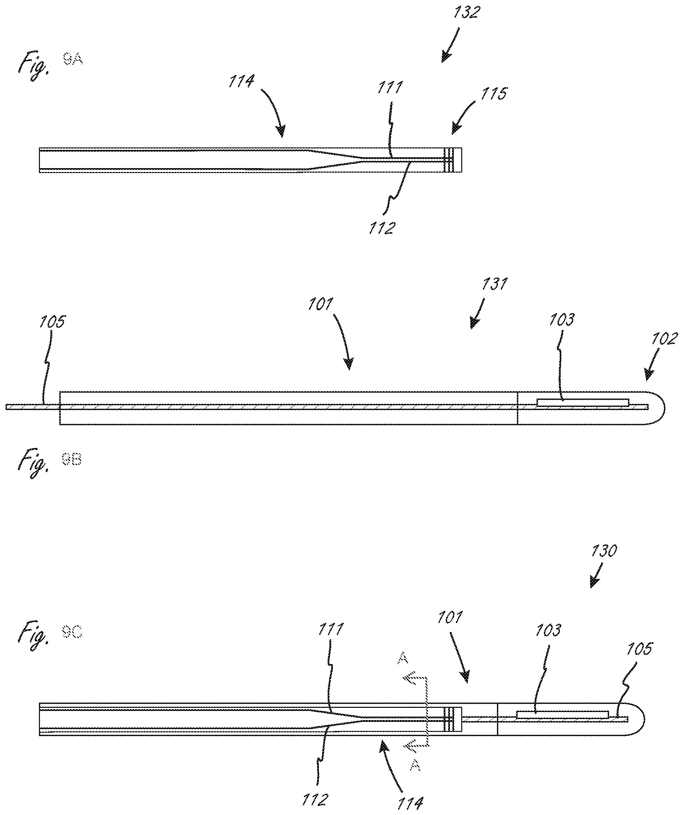

Figure 9C shows exemplary apparatus medical apparatus 130, which includes elongate inner body 132 (see figure 9A) and elongate outer body 131 (see figure 9B).

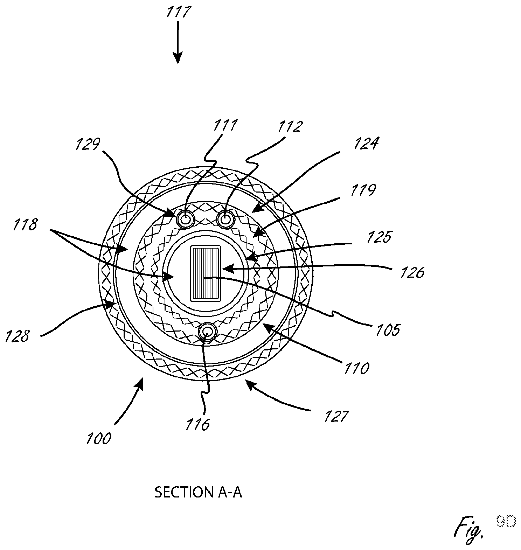

Figure 9D illustrates Section A-A shown in the assembly in figure 9C, which is a section in the deflectable section of the device. Parts from figures 9A-9C are similarly labeled. As can be seen in figure 9D, tensioning members 111 and 112 are very near to one another and about 180 degrees away from straightening tensioning member 116.

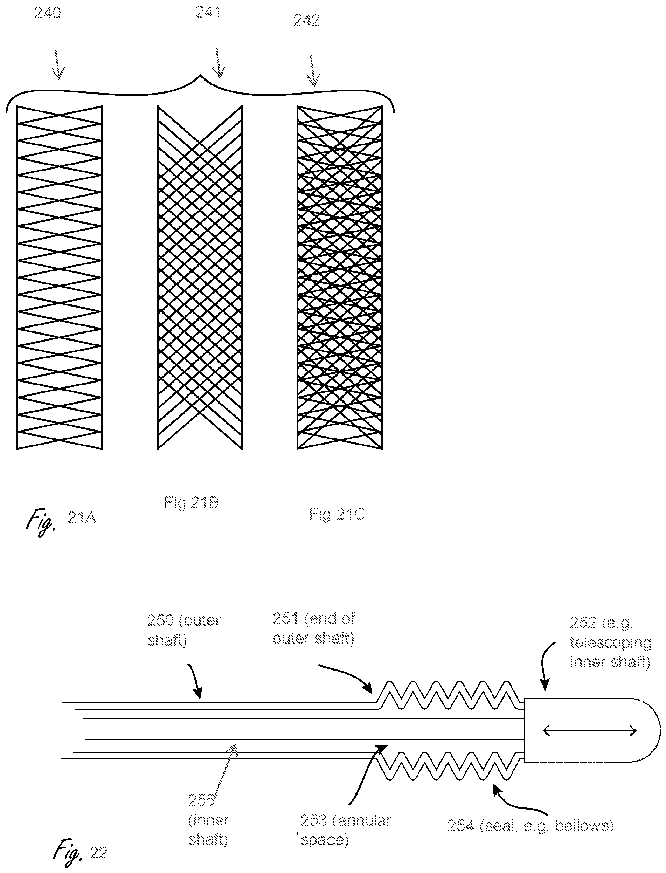

As is also shown in figure 9D, elongate inner body 110 includes two layers of braided material 119, and the tensioning members are, at least at the location of this section, essentially sandwiched between the two layers of braided material. The two layer of braided material canm be the first and second layers in figures 21A-21C, for example. Annular spaces 118 allow freedom of movement and space for optional lubricant. Inner body 110 may be made from, for example without limitation, a polymeric material such as Pebax, optionally with a lubricious additive. Inner body 110 may include liner 125, such as a PTFE liner. The cable bundle 105 may be surrounded by one or more layers of insulation 126, such as PTFE insulation. Outer member 100 may comprise a polymeric material 127, such as Pebax. Outer member 100 may also include a radially inner liner 128, such as a PTFE liner. Any of the pullwires (e.g., 111, 112, 116) may be disposed in a lumen with a liner, such as PTFE liner 129.

Medical apparatus 130 (or either of elongate body 132 and elongate body 131, individually) can be in operable communication with any of the handle assemblies herein, including handle assembly 120 shown in figures 8A and 8B.

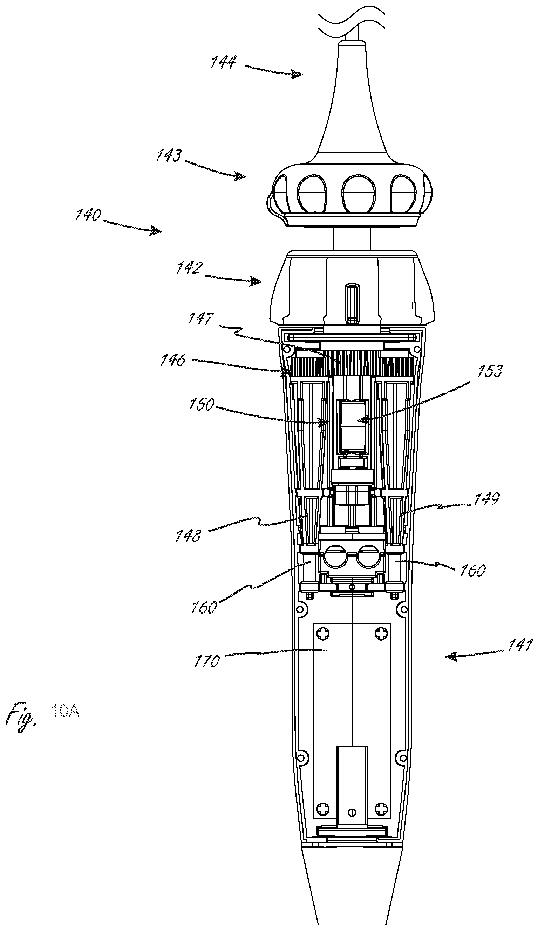

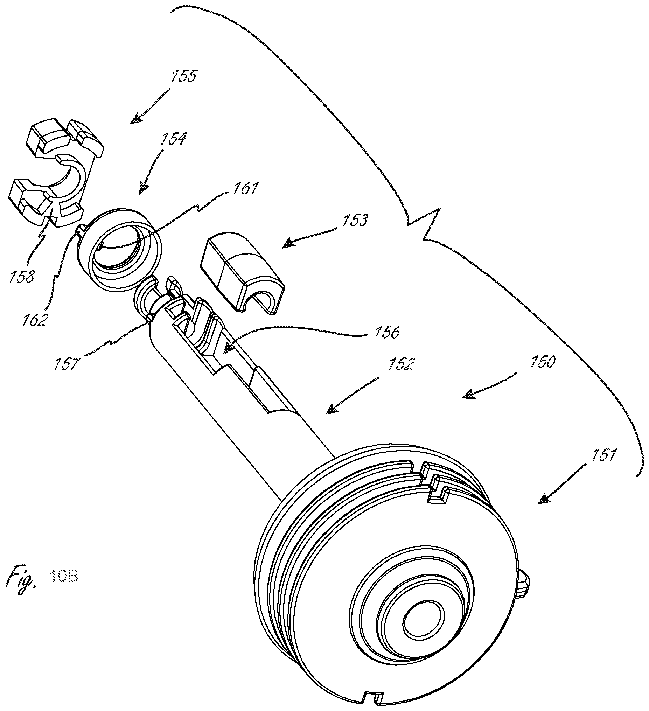

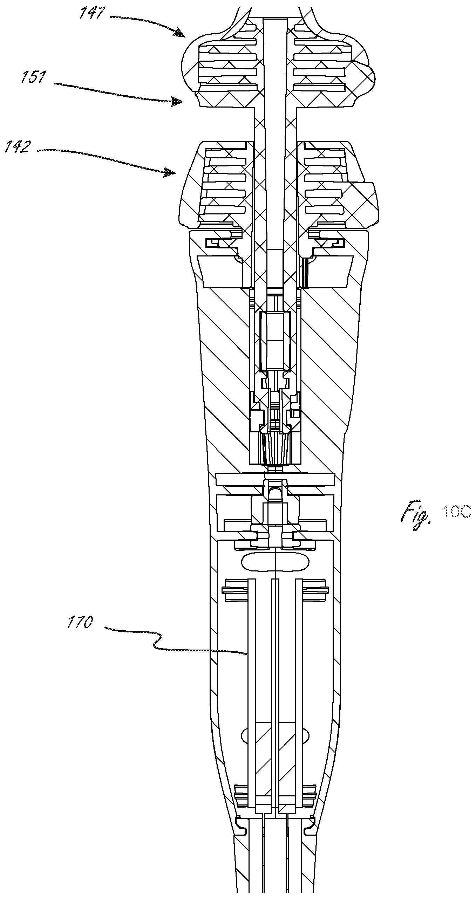

Figures 10A, 10B, 10C, 10D and 10E illustrate an additional exemplary handle assembly that can be in operable communication with any of the medical devices, including ultrasound probes, herein. For example, the exemplary handle assembly shown in figures 10A-10E can be coupled to (directly or indirectly) and in operable communication with medical apparatus 130 shown in figures 9A-9D. In a particular embodiment, both elongate outer body 131 and elongate inner body 132 are coupled to and in operable communication with the handle assembly shown in figures 10A-10E.

The handle assembly in figures 10A-10E has some similarities to the handle assemblies, the individual components, and subassemblies that are shown in figures 8A and 8BB. Unless indicated to the contrary, concepts, features and methods of use from figures 8A and 8B that can be incorporated into the handle assembly in figures 10A-E are hereby incorporated by reference for all purposes into the disclosure of the handle assembly shown in, and described with respect to, figures 10A-E. Similarly, concepts, features and methods of use that are shown in, and described with respect to, figures 10A-E that can be incorporated into other handle assemblies herein are hereby incorporated by reference for all purposes into the disclosure of any of the handle assemblies set forth herein.

Figure 10A is side view of handle assembly 140 with a portion of handle body 141 removed so that some internal components of the handle assembly can be seen. Handle assembly 140 includes first actuator 143 and second actuator 142, and in this embodiment first actuator 143 is distal to second actuator 142. First actuator 143 can be both moved axially and rotated relative to the handle body and relative to a second actuator (in this embodiment actuator 142). First actuator 143 is in operable communication with an outer elongate body, such as outer elongate body 131 (*see* figure 9B). Axial movement of actuator 143 (distally or proximally) causes axial movement of the outer elongate body, while rotation of actuator 143 causes rotation of the outer elongate body. Second actuator 142 is in operable communication with an inner elongate body, such as inner elongate body 132 (*see* figure 9A). Actuation of second actuator 142, in this embodiment rotation, causes deflection of the inner elongate body. In this embodiment a rotatable and axially movable actuator (i.e., first actuator 143) is in operable communication with an outer elongate body.

First actuator 143 is coupled to elongate outer body movement assembly 150 shown in the exploded view in figure 10B, such that movement of first actuator 143 causes movement of assembly 150. Elongate outer body movement assembly 150 is similarly coupled to the elongate outer body so that movement of first actuator also causes movement of the elongate outer body. In this embodiment, the outer elongate body is attached to removable part 153 after it is inserted into channel 156. Removable part 153 and channel 156 are configured so that removable part 153 is constrained by at least one inner surface of channel 156 when it is inserted therein. Elongate outer body movement assembly 150 also includes a distal head portion 151 that is secured to first actuator. Elongate outer body movement assembly 150 also includes a rotation limiting mechanism similar to that which is described herein, which limits the rotation of first actuator 143, and thereby limits the rotation of the outer elongate body. Any of the disclosure above related to rotation limiting subassemblies, functionality, and use, is incorporated into this embodiment for all purposes and may be incorporated into this and similar designs. During rotation, part 157 (see fig. 10B) interacts with part 161, and part 162 interacts with part 158. The physical interactions of these two sets of parts limits rotation to the desired rotation limit, e.g., such as limiting rotation up to 630 degrees of rotation of the outer body (in other embodiments the allowed rotation could be more than 630 degrees, such as up to and including 720 degrees).

If it is desired to clean the outer elongate body, for example after use, removable part 153 can be detached from the outer elongate body to allow the outer elongate body to be removed from the handle assembly and cleaned, before being reinserted and reattached to removable part 153 or a new removable part if part 153 is damaged or broken.

Handle assembly 140 also includes inner elongate body deflection assembly 146, which is in operable communication with second actuator 142. Inner elongate body deflection assembly 146 includes central gear 147 adapted and configured to rotate when second actuator 142 is rotated. Central gear 147 interfaces first spindle 148 and second spindle 149 via a geared interface, such that rotation of central gear 147 causes rotation of the spindles in the opposite direction. The inner elongate body deflection assembly 146, including the spindles, extends further proximally than the elongate outer body movement assembly 150. The inner elongate body extends through the outer elongate body and extends further proximally than the outer elongate body within handle assembly 150. This allows one or more pullwires that are part of the inner elongate body to extend radially outward and interface with reels 160.

The lack of interaction between elongate outer body movement assembly 150 and elongate inner body movement assembly 146 allows for the inner and outer elongate bodies to be independently controlled by first actuator 143 and second actuator 142.

Handle assembly 150 also includes printed circuit board ("PCB") 170 disposed within handle body 141, the PCB being in electrical communication with a cable bundle, such as cable bundle 105 in figure 53, or any of the cable bundles herein that are in communication with the medical tool, such as an ultrasound transducer.

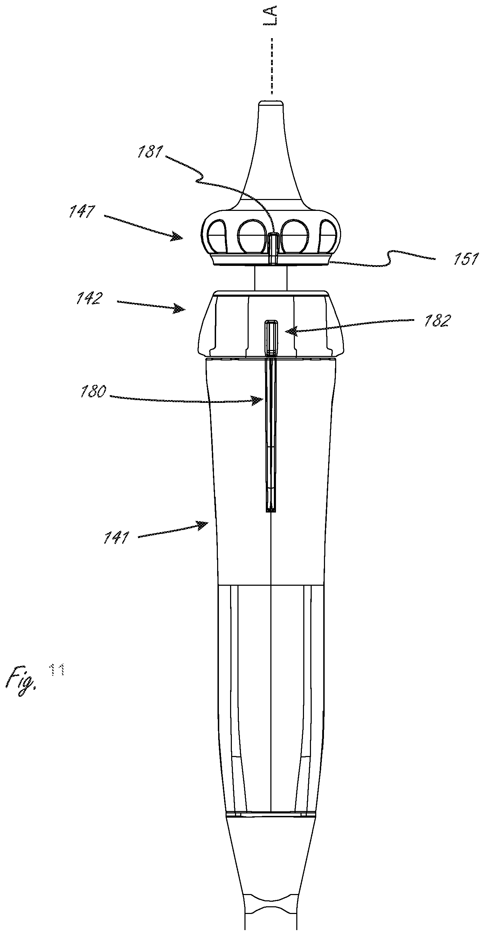

Handle assembly 150 also includes a rotation indicator 180 that can be used to show a user the extent to which at least one of the first actuator and the second actuator are rotated relative to a home, or neutral position. First actuator 143 can include a rotation indicator 181 that is aligned along an axis with rotation indicator 180 when first actuator 143 is in a neutral position, as shown in figure 11. When first actuator 143 is rotated, rotation indicator 181 is rotated relative to the axis along which rotation indicator 180 extends, which enables the user to visually understand that first actuator 143, and thus the elongate outer body, is rotated to some extend relative to the neutral position. Similarly, second actuator 142 can also have rotation indicator 182 that is aligned along an axis with rotation indicator 180 when second actuator 142 is in a neutral position, as shown in figure 11. When second actuator 143 is rotated, rotation indicator 182 is rotated relative to the axis along which rotation indicator 180 extends, which enables the user to visually understand that second actuator 143, and thus the elongate inner body, is deflected to some extent relative to its neutral position.

In some alternative embodiments, the handle assembly can include one or more sensors to track how much rotation has occurred for the outer elongate body, or how much deflection has occurred in the inner elongate body. In some embodiments the handle assembly can include an encoder for each actuator.

In any of the embodiments herein that include an outer elongate body and an inner elongate body, the device can include one or more lubricants between the inner and outer elongate bodies to make it easier to move the inner and outer elongate bodies relative to one another by reducing friction between the two. If the medical device needs to be cleaned for reuse, additional lubricant can be added between the inner and outer elongate bodies after the cleaning process.

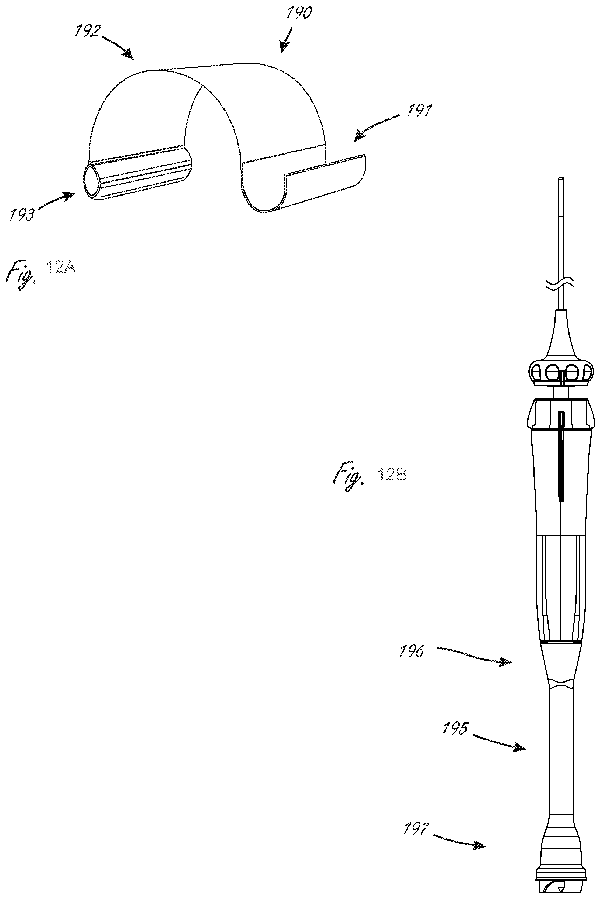

It may be beneficial to be able to stabilize, minimize movement, or maintain the position of a handle of a medical device while it is being used. For example, a physician may not want to have to hold the handle at all times, but may want the handle to stay in a particular position relative to a patient. For example, if a medical tool is an imaging tool (e.g., an ultrasound imaging tool), the physician may not want the tool to rotate or move once it is a desired position, in order to maintain a particular imaging field of view. Currently, a physician may simply place the handle across a portion of the patient's body, but the handle may move once placed down on the patient. Additionally, for example, it may be desirable for a particular medical device to be stabilized when in use, to avoid unwanted movement of one or more distal portions of the medical device. Additionally still, for example, a physician may desire to hold a handle with only one hand, while using the other hand for something else, and may not want the handle to move. Figure 12A illustrates an exemplary handle stabilization member 190 (a handle stabilization member may be also be referred to herein as a handle stabilization apparatus) that can provide any of the functions or benefits just listed, such as at least one of the following, stabilize a medical device handle relative to a part of a patient, minimizing movement of the handle relative to a part of the patient, etc. Handle stabilization member 190 includes handle receiving guide 191 and patient interface region 192, and optional stabilization assist region 193. Guide 191 can be configured with any configuration to allow it to stably interface with a portion of a handle, such as the merely exemplary handle portion 195 in the exemplary handle assembly shown in figure 12B. For example, in this embodiment, guide 191 has a curved configuration to interface with the curved configuration of handle portion 195. In this embodiment the curved configuration is U-shaped, but it could be any other curved shape, such as C-shaped, or any other difficult to define curved configuration. The guide may also be configured with any curvilinear configuration that can stably interface with a portion of the handle. The guide can alternatively have solely flat surfaces (e.g., partial square or partial rectangle).

The guide can be configured to prevent handle movement in at least one direction (e.g., side to side, proximally-distally, etc.). The constraint on handle movement may also depend on the configuration of the handle. For example, guide 191 is configured as a channel, and is adapted to prevent side to side movement while allowing a handle to be lifted upward out of the open region. If a true cylinder handle were placed in guide 191, the cylinder could also move proximally or distally in the guide 191. A handle portion, such as handle portion 195 in figure 12B, can be configured with one or more movement limiting regions 196 and 197 that can further limit or minimize handle movement when interface with the guide 191. For example, movement limiting regions 196 and 197 have larger outer dimensions than the central region in between, so that when handle portion 195 is placed in guide 191 from above, region 197 prevents pure distal movement of the handle, and region 196 prevents pure proximal movement, because of their relatively larger dimensions. The handle could alternatively have only one movement limiting regions, or more than one. The central region between regions 196 and 197, as well as the guide 191, can be sized so that handle portion 195 and guide 191 interface with an interference fit.

Stabilization member 190 includes region or portion 192 that is configured to interface with, and optionally conform to, a portion of the patient. In this embodiment it is envisioned that portion 192 interface with and conform to a patient's leg, but it could have any other configuration so that it interfaces with and conforms to any other surface (e.g., arm, torso, wrist, etc.). Portion 192 can be a rigid material manufactured with the configuration shown, or it can be a flexible material that can more easily conform to a variety of patients. For example, portion 92 could be a polymeric material that has enough stiffness to maintain the position of member 190 when placed on a patient, but flexible enough to be deformed when not in use, such as rolling up to save space in packaging and/or storage. Even if flexible, portion 192 can be manufactured so that it more naturally assumes a configuration of the portion of the patient on which it will be placed. For example, portion 192 can be flexible/foldable, but can have a natural tendency to revert or assume a curved configuration, such as the curved configuration shown in figure 12A. Exemplary configurations of portion 192 include general U-Shape, C-shape, etc, which may facilitate better conformability to legs and arms. Portion 192 can also be rigid and non-deformable, and can be manufactured with any desired configuration.

Stabilizing member 190 also includes optional additional stabilization assist portion 193, which in this exemplary embodiment can act as a counter weight to guide section 191, where the handle will be placed. Stabilization assist portion 193 can have any desired shape, configuration, and/or material that provides the desired weight to counteract the weight of the handle in the guide. Portion 193 is optional and may not be needed. In some embodiments, portion 193 can be made of a different material that provides the counter weight functionality.

In this embodiment, portion 193 is shown on one end of the stabilization member 190, with guide 191 on the other end, and patient interface/conforming portion in between the two.

Figure 12B illustrate an exemplary handle assembly, which is configured to be used with the exemplary handle stabilization member in figure 12A. Other handles can be used with stabilization member 190.

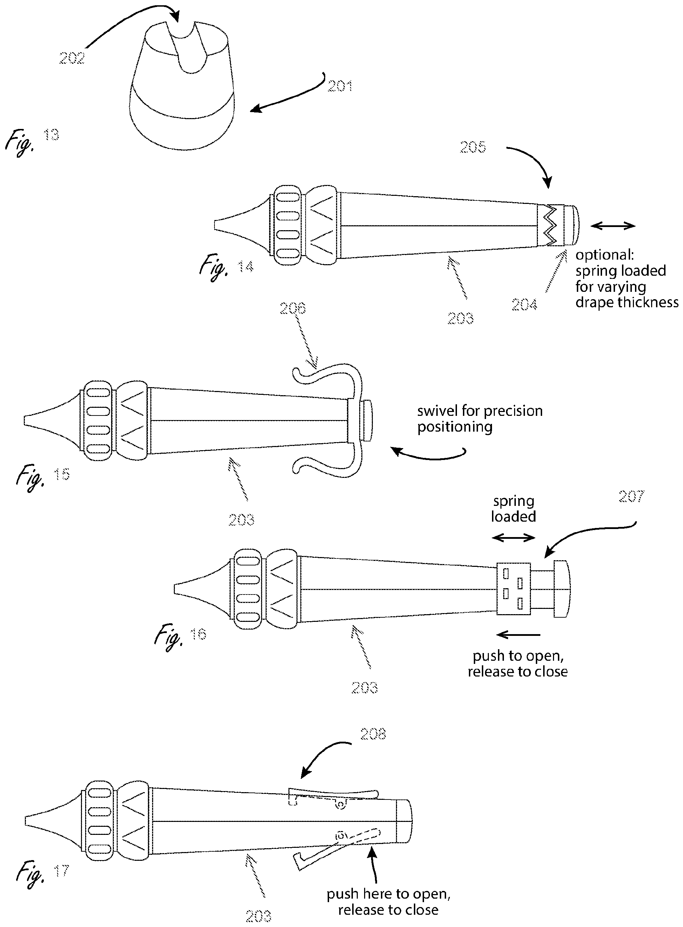

Figure 13 illustrates an exemplary handle stabilizing apparatus that is similar to that shown in figure 12A. The apparatus in figure 13, like the apparatus shown in figure 12A, includes a body member 201 with at least one handle guide 202, the handle guide having at least one surface that is configured to interface with at least a portion of a medical device handle to provide stability to the handle in at least one direction. The handle guide may optionally include magnetic material to help stabilize the handle. The handle guide 202 may optionally include a weighted region at the bottom help stabilize the body member. The body 201 in figure 12 includes an elongate channel guide 202, which is shown. In this exemplary embodiment the channel has a constant width along its length, but in other embodiments the channel may have a width along its length that is not constant, optionally having a greater width at a first channel end than at a second channel end. The optional weighted region (e.g., at the bottom) can bias the body to a natural position on a flat surface so that when its position relative to the flat surface is changed in response to a force, it is biased to return to the natural position when the force it removed. The handle guide or body 201 in figure 13 can include any of the features of any of the handle guides or handle guide regions described herein. In use, the handle guide apparatus in figure 13 or any alternative thereof can be interfaced with a medical device handle to increase the stability of the handle.

The embodiment in figure 13 is an example of a medical device handle stabilizing apparatus, comprising a body 201 including an elongate and recessed handle guide 202, the recessed handle guide including a recessed channel configured to interface with an elongate handle portion of a medical device to provide stability to the handle portion in at least one direction, the body 201 being biased to an upright at-rest position when positioned on a flattened surface, the recessed handle guide facing upward when the body is in the upright position. Body 201 can include a weighted bottom region and a curved bottom surface, the weighted bottom region and the curved bottom surface biasing the body to the upright at-rest position.

A medical device handle can interface with a separate stabilizing apparatus as in figures 12A and 13, although a handle can alternatively or in addition to incorporate one or more features that provide for increased handle stabilization. Figures 14-17 illustrate such exemplary handles. In some embodiments the handle includes a capture mechanism configured and adapted to capture an article that is placed relative to a patient during a medical procedure, optionally wherein the article is an article draped over the patient. Figures 14-17 illustrate exemplary handles that include one or more capture mechanisms. Figure 14 illustrates a capture mechanism that includes first and second portions that are configured to interface with the article and capture the article between the first and second portions. The second portion in this embodiment in movable relative to the first portion, and can optionally be biased to return towards a home position, such as by being spring-loaded.

Figure 15 illustrates first and second capture mechanisms of a handle, e.g., two clips which may be adapted to swivel relative to the main handle body.

The one or more capture mechanisms can be disposed on a proximal portion of the handle, such as in figures 14-17.

The embodiments in figures 14-17 are examples of a medical device, the medical device including an elongate handle housing 203, and an object capturing member (e.g., 205, 206, 207, 208) secured relative to the handle housing, the object capturing member including a movable member (e.g., 204, 206) that is movable relative to the handle housing from an open position to a closed position, wherein in the closed position (such as shown in figure 14) the movable member is closer to a first portion of the handle than in the open position. In figure 14, object capturing member 205 comprises flexible teeth adapted for capturing an article of clothing. The moveable member (e.g. 204, 208, 207) may optionally be spring loaded (biased), such as for varying thickness of an article of clothing or garment. The movable member can be biased to return towards the closed position from the open position. The moveable member can be secured relative to the handle housing such that the movable member moves axially along the handle housing when it is moved from the closed position to the open position. The moveable member (e.g. 206) can be secured relative to the handle housing such that the moveable member is moved radially outward from the handle housing when moving from the closed position to the open position. The medical device handle can have a length, and wherein the object capturing member (e.g. 205, 206, 207, 208) is disposed in a proximal half of the handle. Object capturing member 206 includes first and second clips extending in different radially directions relative to handle housing 203, portions of each of which can be deformed radially outward to an open configuration. The moveable member (e.g. 206, 208) may comprise first and second movable members. The handles in figures 14-17 can be coupled to any of the elongate catheters herein, including any that include an ultrasound imaging element disposed at a distal end. The object capturing member (e.g., 206) can be adapted to rotate relative to the elongate handle housing, as shown in exemplary figure 15. The medical device handle can include one or more medical device control actuators, and wherein the one or more medical device control actuators are disposed in a distal half of medical device handle. Figures 14-17 includes first and second control actuators, which can operate in any of manners described herein to control, for example, inner and outer shafts.

Figures 18 and 19 illustrate exemplary handles 203 that include one or more features adapted and configured for increased handle stabilization. Figures 18 and 19 illustrate handles 203 that includes at least one moveable extension 210 and 211, respectively (two are included in figure 18), the movable extension movable relative to a body portion of the handle. At least a portion of at least one of the at least one movable extension extends away from a longitudinal axis of the handle. The movable extension can extend away from a longitudinal axis as well as be reconfigurable, such as in figure 18. At least one of the at least one movable extension (e.g. 211) can be rotatable relative to the body portion, such as in figure 19. At least one of the at least one movable extension can be adapted to be reconfigured (e.g. figure 18), optionally at one or more locations, and further optionally along one or more discrete locations, including any feature of flexible arms of a flexible tripod.

Figures 18 and 19 are examples of medical devices that comprise elongate handle housing 203 and a movable handle housing stabilizing member 210 (two are shown) and 211, respectively, that extends away from the elongate handle housing, the stabilizing member having at least one surface that is adapted to be moved relative to the elongate handle housing to interact with an adjacent object (e.g. a table) and stabilize the elongate handle housing relative to the adjacent object. Figure 19 is an example of a movable handle housing stabilizing member (e.g. 211) that extends radially away from the elongate handle housing. Figure 19 is an example of a movable handle housing stabilizing member that includes first and second radial projections extending radially away from the handle housing in different radial directions, optionally extending radially away 180 degrees from each other. Figure 18 is an example of a movable handle housing stabilizing member that includes one or more arms (e.g. 210) extending form the handle housing 203, the arms adapted to be reconfigured at one or more locations along their lengths to stabilize the handle housing relative to an adjacent object. Figure 18 is also an example of first and second arms (e.g. 210) each have a plurality of linkages (shown figure 18) to facilitate bending at each link.

Some medical procedures include viewing one or more images being displayed on a display, which may be part of a console. In some procedures there is a sterile field, in which a medical practitioner is located, and the display and/or console are outside of the sterile field. The medical practitioner must stay within the sterile field, but may want to interface with the display and/or console outside of the sterile field, and thus generally has to request a technician outside of the sterile field to interact directly with the display and/or console to cause some event to occur, such as changing a view shown on the display (e.g., rotating a view), or changing to a different view, for example.

One aspect of the disclosure provides the medical practitioner inside the sterile field with the ability to directly control one or more aspects of the display and/or console. Any of the handles herein can include one or more controllers, which are adapted to allow medical personnel to interact with them to cause some event to occur on a display and/or in a console. A controller may be one or more of a tactile controller (i.e., adapted to be responsive to touch (e.g., buttons, sliders, etc.)) and an audio controller (e.g., responsive to voice commands). A controller can be in electrical communication with a cable extending from the handle. An example of a tactile controller is a button, which can be pushed to cause an event to take place.

Software (computer executable methods), such as a software stored on a console, can be adapted to respond to actuation of a controller to cause an event to occur. For example, software can cause a change in a displayed image (e.g., an ultrasound image) in response to actuation of the handle controller. Software can be adapted to cause a new image to be displayed on a display in response to actuation of a controller.

Images that can be stored can be real-time images obtained using an imaging tool, or they may be stored images. For example, images may be biplane or triplane images, or 3D volumes.

Figure 20 illustrates an example of a medical device, comprising an elongate handle housing 203; and one or more actuatable inputs (generally shown as 220) that are in operative communication with a cable 221 extending proximally from the elongate handle housing, a proximal region of the cable including a coupler that is adapted to be coupled to an external imaging console. While a coupler is not shown in figure 20, an example is shown in figure 7. Figure 20 is an example of one or more actuatable inputs that includes at least one of a tactile actuatable input or an actuatable audio input. In the exemplary figure 21, four pushable arrow buttons are included, as are 4 separate pushable buttons, which may have particular functions for controlling one or more aspects of a display on an external console, such as panning through a variety of images, such as images with different viewing angles.

The disclosure also includes computer executable methods that can be stored on an external medical imaging console, the methods including receiving as input information indicative of an actuation of an input on an elongate medical device handle housing (e.g., 203 in figure 20), and causing an image being displayed on the external medical imaging console to be modified in one or more ways. The modification can include at least one of a new image to be displayed, a change in magnification or an annotation being added to an existing figure. The modification can include displaying a different view of a region of a subject's body. The embodiment in figure 42 can be configured to be an elongate medical device handle housing that includes a plurality of actuatable inputs, wherein the modification includes displaying a particular image view that is pre-selected based on actuation of a first of the plurality of actuatable inputs (e.g., any of the buttons shown in figure 20), wherein actuation of the first input is associated with the particular image view and causes the particular image view to be displayed.

An aspect of the disclosure is a medical device shaft that includes first and second reinforcing structure (e.g., braided material), one radially within the other, wherein the first reinforcing structure is better adapted for torque response than the second reinforcing structure, and wherein the second reinforcing structure is better adapted for kink resistance than the first reinforcing structure. The medical shaft with the reinforcing structures can be used for any of the inner and outer shafts described herein.

In some embodiments the reinforcing structures are braided materials. Figures 21A-C illustrates first and second exemplary braided materials, with one radially within the other, which optionally may be implemented in a device such as shown in figure 9D. The rest of the shaft is not shown for clarity. Reinforcing structure 240 has is better adapted for torque response than reinforcing structure 241, while reinforcing structure 241 is better adapted for kink resistance than reinforcing structure 240. The combination of the two reinforcing structures 242 provides for good pushability and good torque transmission, while retaining flexibility and low force to deflect. The first reinforcing structure 240 can be inside or outside of the second reinforcing structure 241.

In the exemplary embodiment in figures 21A-C, braid layer 240 has a first picks per inch ("PPI") (1 inch = 25.4mm) the second braid layer 241 has a second PPI, the first PPI being different than the second PPI. In the embodiment in figures 21A-C, the first PPI is lower than the second PPI, and the first reinforcing structure 240 is radially within the second reinforcing structure 241. In other embodiments, the first PPI can be greater than the second PPI, and the first reinforcing structure can be outside the second reinforcing structure.

The shallower angle of reinforcing structure 241 prevents the shaft from being compressed too much, whereas the steeper angle of reinforcing structure 240 would allow more compression by itself.

The first and second reinforcing structures can be embedded in a polymeric material, using common general manufacturing techniques.

The medical shaft can be adapted to be deflected, steered, bent, or any other term used to change the configuration of the shaft. The shaft can have one or more pullwires disposed between the first and second reinforcing structures to cause the deflection in any number of directions.

The reinforcing structures can extend along any desired length of portion of the shaft as desired, such as at least within a portion that is adapted to be deflected, steered, bent, etc.

There isn't an exact angle at which the braids need to be formed; they can differ and still provide the functionality provided herein. Similarly, the PPI need not be an exact number; they can differ and still provide the functionality provided herein.

One or more pull wires are interweaved in the outer reinforcing structure, the inner reinforcing structure, or both. An example of this is shown in the embodiment in figure 9D.

In any of the embodiments, a flat material can be used for the inner braid; and the outer braid is a non-flat material, such as a round material. This can help reduce the size of the inner reinforcing structure. The inner braid can be flat or round, and the outer braid can be flat or round.

The embodiment in figs 21A-C (as well as figure 9D) is an example of first and second braided layers that can be incorporated into an elongate medical device. The elongate medical device can be sized and configured for intravascular delivery within a subject, comprising: a deflectable shaft extending distally from a handle portion of the medical device, the deflectable shaft including first and second braided layers (e.g. 240, 241) each of which includes one or more strands (as shown in figures 21A-C), one of the first and second braided layer disposed radially within the other and co-axial with the other, and a flexible polymeric layer, the first and second braided layers at least partially embedded in the flexible polymer layers, the first braided layer having a first strand construction and the second braided layer having a second strand construction such that the first braided layer is better at transmitting torque alone than the second braided layer alone, and such that second braided layer is more resistant to kinking alone than the first braided layer alone, the flexible shaft (such as a shaft that includes 242) better at transmitting torque compared to a second flexible shaft with the second braided layer and without the first braided layer, the flexible shaft more resistant to kinking than a third flexible shaft with the first braided layer but without the second braided layer. According to the invention, the elongate medical device includes one or more pullwires, such as in the embodiment in figure 9D. The pullwires are at least partially interwoven in at least one of the first braided layer or the second braided layer.

One aspect of the disclosure is a medical device comprising an outer shaft and an inner shaft, the outer and inner shafts axially movable relative to one another; and a flexible seal disposed to seal off the ingress of fluid into a space between the outer shaft and the inner shaft. Some medical devices, when in use, may allow for blood to flow in between two shaft. Figures 22-26 illustrate medical devices that can prevent blood to enter into the space between shafts. The seal may be secured (directly or indirectly) to at least one of the inner and outer shafts, and can extend outside of at least a portion of the inner shaft to seal off from the ingress of fluid into a space between the outer shaft and the inner shaft. Rotation limiters are described herein, which prevents a seal being rotated too much and possible tearing.

Figure 22 is a side view of a distal region of a medical device that includes an outer shaft and an inner shaft, at least one of which can be moved relative to the other using any desired handle construction. The medical device includes a flexible seal 254, in this embodiment in the form of bellows to allow for the relative axial movement, and optionally for relative rotational movement. The seal in this embodiment is coupled directly to the outer shaft 250 and to a tip portion 252 (e.g., comprising an ultrasound transducer) that is axial affixed to the inner shaft 255. The seal can be made of material that allows it be moved axially and optionally rotationally as well.

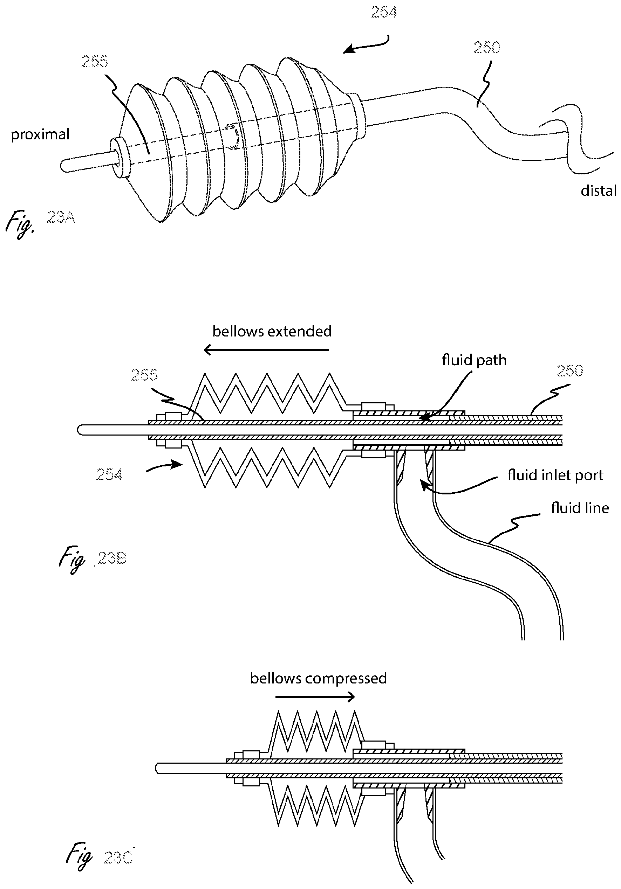

Figures 23A-26 illustrate medical devices with flexible seals 254 at a proximal region of the device. Similar parts may be so identified by similar reference numbers. Any description above with seals at the distal shaft can be integrated into seals at the proximal region. In figures 23A-C, the flexible seal 254 is coupled to at least one of an inner shaft or an outer shaft, but need not be directly coupled thereto, but is axially affixed relative thereto.

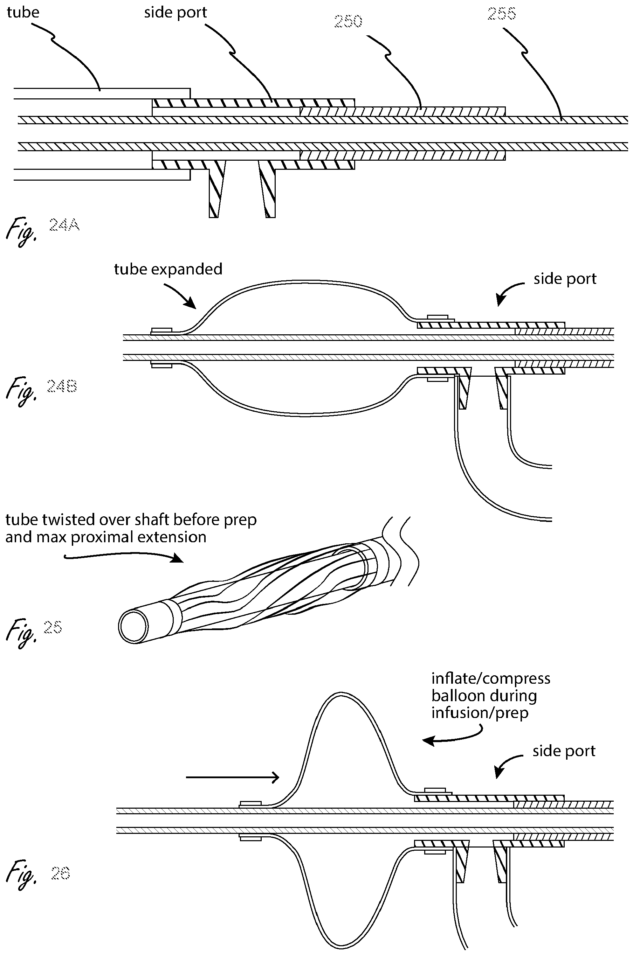

Any of the flexible seals herein can assume any configuration, such as bellows, a flexible bag, balloon, etc. A flexible seal can be adapted to be inflated and can be in fluid communication with a fluid source, such as in figure 26. Any of the flexible seals herein can be constructed to be able to allow for at least one of axial movement and rotational movement of the flexible seal.

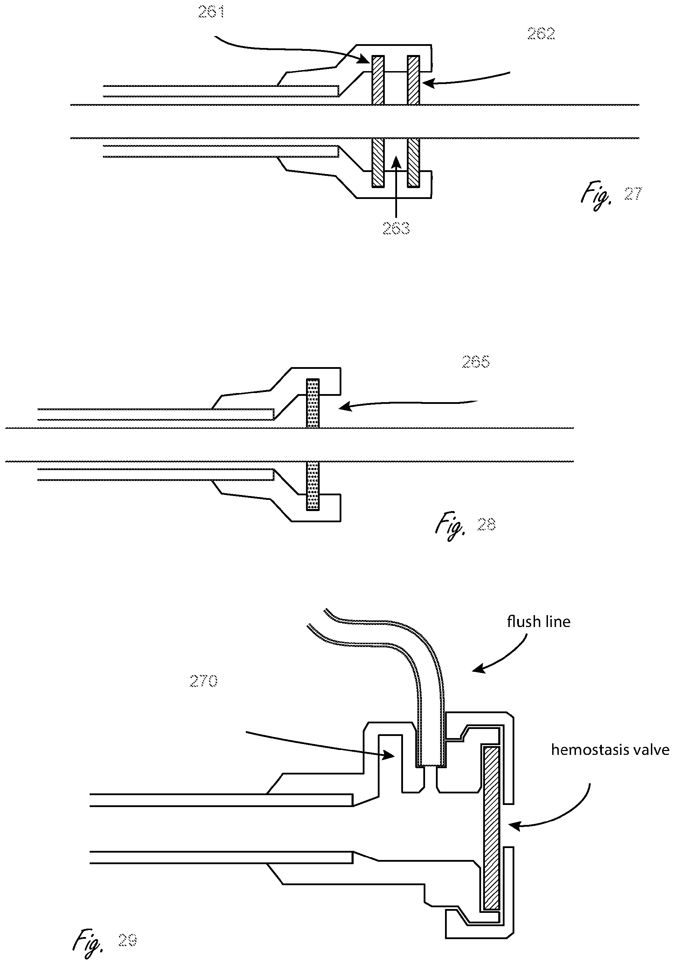

One aspect of the disclosure is related to hemostasis valves, some of the designs of which may present challenges when trying to clean them. Figures 27-29 are such examples. In figure 27, there are two seals, which is easier to sterilize but harder to clean. The second seal may be an ePTFE seal, whereas the first seal can be a common valve.

Figures 27 and 28 are examples of hemostasis valves for a medical device, comprising: a seal constructed of a material into which bodily fluid can penetrate when they come into contact with the seal, the seal interfacing an axially movable elongate shaft of the medical device. In Figure 28, the seal may be at least partially an open cell material such as foam. An inner edge of the seal could also be coated with silicone. The rest of the medical device is not shown for clarity.

Figure 27 shows a device with a common hemostasis valve 261, an ePTFE seal 262 that is adapted to allow gas in and out but not fluid, and a dead-end void 263 created by the two seal that gas can enter but more challenging for fluid and other materials.

In figure 28, a proximal region of the medical device includes an open cell foam or ePTFE or similar material seal 265 that body fluid can penetrate, but is difficult to clean.

Figure 29 is an example of a proximal region of a medical device that includes a recess 270 that is difficult to clear and clean. Other exemplary components are labeled.

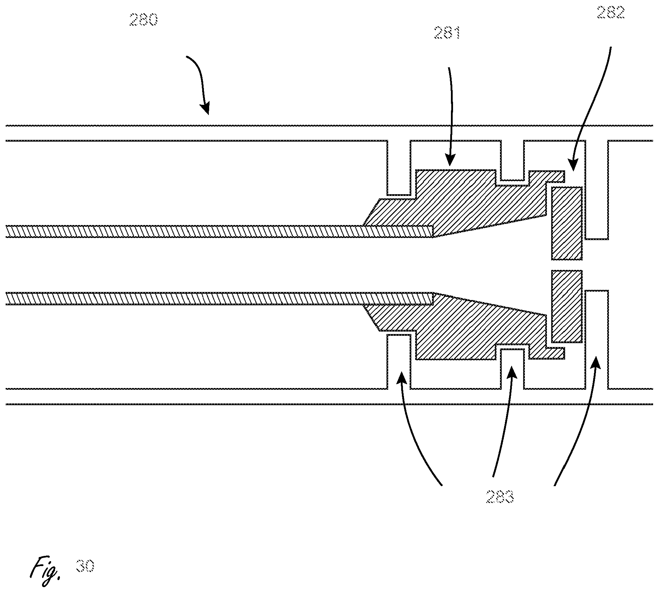

Figure 30 illustrates a proximal portion of a medical device that includes handle housing or shell 280, hemostasis valve body 281, hemostasis valve sheet 282, and a plurality of handle shell protrusions 283 (extending radially inward) that hold hemostasis valve body and sheet in place and create compression to make a tight hemostasis seal. This design eliminates the need for a valve cap.

An aspect of the disclosure is a portion of a handle is difficult to clean to control reprocessing. Figure 29 is such an example, with a dead end space that would be difficult to clean, which would likely require a new part to be made before the device could be reused. The handle can also have a filter on top so air can be released and not get trapped. Figure 29 shows a handle of a medical device, wherein an open chamber formed internally in a portion of the handle has at least one dimension that is no greater than 1cm, optionally no greater than 9 mm, optionally no greater than 8 mm, optionally no greater than 7 mm, optionally no greater than 6 mm, optionally no greater than 5 mm, optionally no greater than 4 mm, optionally no greater than 3 mm, optionally no greater than 2 mm.

Figure 30 illustrates a medical device handle outer shell (the body positioned to be held by a user), comprising a plurality of inner features formed in the shell, each of which is sized and configured to interface with at least one of a hemostasis valve body and a hemostasis valve sheet, such that the plurality of shell features, when the handle is assembled, hold the hemostasis valve body and the hemostasis valve sheet in place relative to one another and create compression to create a seal.

## Claims

1. An elongate medical device (1000) sized and configured for intravascular delivery within a subject, comprising:
a deflectable shaft (1004) extending distally from a handle portion of the medical device, the deflectable shaft including:
first and second braided layers (240, 241) each of which includes one or more strands, one of the first and second braided layer disposed radially within the other and co-axial with the other;
a flexible polymeric layer, the first and second braided layers at least partially embedded in the flexible polymer layer; and
one or more pullwires interweaved in at least one of the first braided layer or the second braided layer,
the first braided layer having a first strand construction and the second braided layer having a second strand construction such that the first braided layer is better at transmitting torque alone than the second braided layer alone, and such that second braided layer is more resistant to kinking alone than the first braided layer alone.

2. The medical of claim 1, wherein the first strand construction has a first picks per inch ("PPI") that is different than a second picks per inch of the second strand construction (1 inch = 25.4 mm).

3. The medical device of claim 1, a first of the one or more pullwires disposed on a first side of the deflectable shaft, and a second of the one or more pullwires disposed on a second side of the deflectable shaft at least 135 degrees away from the first pullwire.

4. The medical device of claim 1, wherein the deflectable shaft is better at transmitting torque compared to a second flexible shaft with the second braided layer and without the first braided layer, the deflectable shaft is more resistant to kinking than a third flexible shaft with the first braided layer but without the second braided layer.

## Patentansprüche

1. Längliche medizinische Vorrichtung (1000), die für die intravaskuläre Zuführung in einem Patienten bemessen und ausgestaltet ist, umfassend:
einen auslenkbaren Schaft (1004), der sich distal von einem Griffabschnitt der medizinischen Vorrichtung erstreckt, wobei der auslenkbare Schaft Folgendes aufweist:
eine erste und eine zweite geflochtene Lage (240, 241), die jeweils einen oder mehrere Stränge aufweisen, wobei eine der ersten und der zweiten geflochtenen Lage radial in der anderen und koaxial zu der anderen angeordnet ist, eine flexible polymere Lage, wobei die erste und die zweite geflochtene Lage mindestens teilweise in der flexiblen Polymerlage eingebettet sind, und
einen oder mehrere Zugdrähte, die in der ersten geflochtenen Lage und/oder der zweiten geflochtenen Lage verwoben sind,
wobei die erste geflochtene Lage eine erste Strangkonstruktion hat und die zweite geflochtene Lage eine zweite Strangkonstruktion hat, so dass die erste geflochtene Lage besser ist, wenn Drehmoment allein überträgt, als wenn die zweite geflochtene Lage Drehmoment allein überträgt, und so dass die zweite geflochtene Lage allein knickfester ist als die erste geflochtene Lage allein.

2. Medizinische nach Anspruch 1, wobei die erste Strangkonstruktion eine erste Flechtdichte (PPI - picks per inch) hat, die von einer zweiten Flechtdichte der zweiten Strangkonstruktion verschieden ist (1 Zoll = 25,4 mm) .

3. Medizinische Vorrichtung nach Anspruch 1, wobei ein erster des einen oder der mehreren Zugdrähte an einer ersten Seite des auslenkbaren Schafts angeordnet ist und ein zweiter des einen oder der mehreren Zugdrähte an einer zweiten Seite des auslenkbaren Schafts mindestens 135 Grad von dem ersten Zugdraht weg angeordnet ist.

4. Medizinische Vorrichtung nach Anspruch 1, wobei der auslenkbare Schaft beim Übertragen von Drehmoment im Vergleich zu einem zweiten flexiblen Schaft mit der zweiten geflochtenen Lage und ohne die erste geflochtene Lage besser ist, wobei der auslenkbare Schaft knickfester ist als ein dritter flexibler Schaft mit der ersten geflochtenen Lage, aber ohne die zweite geflochtene Lage.

## Revendications

1. Dispositif médical allongé (1000) dimensionné et conçu pour une pose intravasculaire à l'intérieur d'un sujet, comprenant :
un arbre fléchissable (1004) s'étendant dans le sens distal à partir d'une partie formant poignée du dispositif médical, l'arbre fléchissable comportant :
des première et deuxième couches tressées (240, 241) dont chacune comporte un ou plusieurs filaments, l'une des première et deuxième couches tressées étant disposée radialement à l'intérieur de l'autre et coaxiale par rapport à l'autre ;
une couche polymérique flexible, les première et deuxième couches tressées étant au moins partiellement incorporées dans la couche polymérique flexible ; et
un ou plusieurs fils de traction entremêlés dans au moins l'une parmi la première couche tressée et la deuxième couche tressée,
la première couche tressée ayant une première construction de filament et la deuxième couche tressée ayant une deuxième construction de filament de sorte que la première couche tressée transmet mieux le couple seule que la deuxième couche tressée seule, et de sorte que la deuxième couche tressée est plus résistante à la coudure seule que la première couche tressée seule.

2. Médical selon la revendication 1, la première construction de filament ayant un premier nombre de duites par pouce (PPI) différent d'un deuxième nombre de duites par pouce de la deuxième construction de filament (1 pouce = 25,4 mm).

3. Dispositif médical selon la revendication 1, un premier des un ou plusieurs fils de traction étant disposé sur un premier côté de l'arbre fléchissable, et un deuxième des un ou plusieurs fils de traction étant disposé sur un deuxième côté de l'arbre fléchissable au moins 135 degrés à l'écart du premier fil de traction.

4. Dispositif médical selon la revendication 1, l'arbre fléchissable transmettant mieux le couple par comparaison avec un deuxième arbre flexible avec la deuxième couche tressée et sans la première couche tressée, l'arbre fléchissable étant plus résistant à la coudure qu'un troisième arbre flexible avec la première couche tressée mais sans la deuxième couche tressée.
